# DEMANDE DE BREVET EUROPEEN

(11) **EP 1 523 992 A1**
(43) Date de publication de la demande: **20.04.2005**
(21) Numéro de dépôt: 04021994.1
(22) Date de dépôt: 16.07.1997
(51) Int. Cl.: A61K 39/12

(54) **Formules de vaccin contre la maladie de Gumboro**

(30) Priorité: 19.07.1996 FR 9609339
(62) Demande divisionnaire de: 97934579.0
(71) Demandeur: MERIAL, 69002 Lyon (FR)
(72) Inventeur: Audonnet, Jean-Christophe, 69006 Lyon (FR); Bouchardon, Annabelle, 69007 Lyon (FR); Riviere, Michel, 69130 Ecully (FR)
(74) Mandataire: Nargolwalla, Cyra

(57) **Abrégé**

La présente invention concerne des formules de vaccin aviaire contre la maladie de Gumboro.

SEQUENCE LISTING

<110> Merial

<120> Formules de vaccins contre la maladie de Gumboro

<130> GBu/CN - 04021994

<140> EP 04 021 994. 1
<141> 2004-09-16

<160> 44

<170> PatentIn version 3.3

<210> 1
<211> 37
<212> DNA
<213> Artificial

<220>
<223> oligonucleotide as PCR primer

<400> 1
aaaactgcag actatgcact attttaggcg gaattgc 37

<210> 2
<211> 35
<212> DNA
<213> Artificial

<220>
<223> oligonucleotide as PCR primer

<400> 2
ggaagatctt tacacagcat catcttctga gtctg 35

<210> 3
<211> 29
<212> DNA
<213> Artificial

<220>
<223> oligonucleotide as PCR primer

<400> 3
   aaactgcaga tgaaagtatt tttttttag 29

<210> 4
<211> 32
<212> DNA
<213> Artificial

<220>
<223> oligonucleotide as PCR primer

<400> 4
ggaagatctt tataggcggg aatatgcccg tc 32

<210> 5
<211> 39
<212> DNA
<213> Artificial

<220>
<223> oligonucleotide as PCR primer

<400> 5
ataagaatgc ggccgccatg gaccgtgcag ttagcagag 39

<210> 6

<211> 34
<212> DNA
<213> Artificial

<220>
<223> oligonucleotide as PCR primer

<400> 6
cgcggatcct taaatcccat catccttgag aatc 34

<210> 7
<211> 1716
<212> DNA
<213> Newcastle disease virus

<400> 7
atggaccgtg cagttagcag agttgcgcta gagaatgaag aaagagaagc aaagaataca 60

tggcgctttg tattccggat tgcaatctta cttttaatag taacaacctt agccatctct 120

gcaaccgccc tggtatatag catggaggct agcacgcctg gcgaccttgt tggcataccg 180

actatgatct ctaaggcaga agaaaagatt acatctgcac tcagttctaa tcaagatgta 240

gtagatagga tatataagca ggtggccctt gagtctccat tggcgttgct aaacactgaa 300

tctgtaatta tgaatgcaat aacgtctctc tcttatcaaa tcaatggagc tgcaaataat 360

agcgggtgtg gggcacctgt tcatgaccca gattatatcg gggggatagg caaagaactt 420

attgtggatg acgctagtga tgtcacatca ttctatccct ctgcgttcca agaacacctg 480

aactttatcc cggcacctac tacaggatca ggttgcactc ggataccctc attcgacata 540

agcgctaccc actactgtta cactcacaat gtgatattat ctggttgcag agatcactca 600

cactcatatc agtacttagc acttggcgtg cttcggacat ctgcaacagg gagggtattc 660

ttttctactc tgcgttccat caatttggat gacagccaaa atcggaagtc ttgcagtgtg 720

agtgcaactc ccttaggttg tgatatgctg tgctctaaaa tcacagagac tgaggaagag 780

gattatagtt caattacgcc tacatcgatg gtgcacggaa ggttagggtt tgacggtcaa 840

taccatgaga aggacttaga cgtcataact ttatttaagg attgggtggc aaattaccca 900

ggagtggggg gtgggtcttt tattaacaac cgcgtatggt tcccagtcta cggagggcta 960

aaacccaatt cgcctagtga caccgcacaa gaagggagat atgtaatata caagcgctac 1020

aatgacacat gcccagatga acaagattac cagattcgga tggctaagtc ttcatataag 1080

cctgggcggt ttggtggaaa acgcgtacag caggccatct tatctatcaa ggtgtcaaca 1140

tctttgggcg aggacccggt gctgactgta ccgcctaata caatcacact catgggggcc 1200

gaacggagag ttctcacagt agggacatct catttcttgt accagcgagg gtcttcatac 1260

ttctctcctg ctttattata ccctatgaca gtcaacaaca aaacggctac tcttcatagt 1320

ccttacacat tcaatgcttt cactaggcca ggtagtgtcc cttgtcaggc atcagcaaga 1380

tgccccaact catgtgtcac tggagtttat actgatccgt atcccttagt cttccatagg 1440

aaccatacct tgcggggggt attcgggaca atgcttgatg atgaacaagc aagacttaac 1500

cctgtatctg cagtatttga taacatatcc cgcagtcgca taacccgggt aagttcaagc 1560

cgtactaagg cagcatacac gacatcgaca tgttttaaag ttgtcaagac caataaaaca 1620

tattgcctca gcattgcaga aatatccaat accctcttcg gggaattcag gatcgttcct 1680

ttactagttg agattctcaa ggatgatggg atttaa 1716

<210> 8
<211> 37
<212> DNA
<213> Artificial

<220>
<223> oligonucleotide as PCR primer

<400> 8
agaatgcggc cgcgatgggc tccagatctt ctaccag 37


<210> 9
<211> 34
<212> DNA
<213> Artificial

<220>
<223> oligonucleotide as PCR primer

<400> 9
tgctctagat catatttttg tagtggctct catc 34


<210> 10
<211> 1662
<212> DNA
<213> Newcastle disease virus

<400> 10
atgggctcca gatcttctac caggatcccg gtacctctaa tgctgatcat ccgaaccgcg 60

ctgacactga gctgtatccg tctgacaagc tctcttgatg gcaggcctct tgcggctgca 120

gggatcgtgg taacaggaga taaagcagtc aacatataca cctcatccca gacagggtca 180

atcatagtta agttactccc gaatatgccc aaggacaaag aggtgtgtgc aaaagcccca 240

ttggaggcat acaacaggac actgactact ttactcaccc cccttggtga ttctatccgc 300

aggatacaag agtctgtgac tacttccgga ggaaggagac agagacgctt tataggtgcc 360

attatcggca gtgtagctct tggggttgcg acagctgcac agataacagc agcttcggcc 420

ctgatacaag ccaaccagaa tgctgccaac atcctccggc ttaaagagag cattgctgca 480

accaatgaag ctgtgcacga ggtcactgac ggattatcac aactagcagt ggcagtaggg 540

aagatgcaac agtttgtcaa tgaccagttc aataatacag cgcaagaatt ggactgtata 600

aaaattgcac agcaggtcgg tgtagaactc aacttgtacc taactgaatt gactacagta 660

tttgggccac aaatcacttc ccctgcctta actcagctga ctatccaagc gctttacaat 720

ctagctggtg gtaatatgga ttacttgctg actaagttag gtgtagggaa caaccaactc 780

agctcattaa ttggtagcgg cttgatcacc ggcaacccta ttctgtacga ctcacagact 840

cagatcttgg gtatacaggt aactttgcct tcagttggga acctgaataa tatgcgtgcc 900

acctacctgg agaccttatc tgtaagcaca accaagggat ttgcctcagc acttgtccca 960

aaagtggtga cacaggtcgg ttccgtgata gaagaacttg acacctcata ctgtataggg 1020

accgacttgg atttatactg tacaagaata gtgacattcc ctatgtctcc tggtatttat 1080

tcttgtctga gcggtaatac atcggcttgc atgtattcaa agactgaagg cgcacttact 1140

acgccatata tggctctcaa aggctcagtt attgccaatt gcaagctgac aacatgtaga 1200

tgtgcagatc ccccaggtat catatcgcaa aattatggag aagctgtgtc cttaatagat 1260

aggcactcat gcaacgtctt atccttagac gggataactc tgaggctcag tggggaattt 1320

gatgcaacct atcaaaagaa tatctctata ctagattctc aagttatagt gacaggcaat 1380

cttgatatat caactgagct tgggaatgtc aacaactcaa taagtaatgc cctgaataag 1440

ttagaggaaa gcaacagcaa actagacaaa gtcaatgtca aactgaccag cacatctgct 1500

ctcattacct acatcgtttt aactgtcata tctcttgttt ttggtgtact tagcctggtt 1560

ctagcatgct acctgatgta caagcaaaag gcacaacaaa agaccttgtt atggcttggg 1620

aataataccc ttgatcagat gagagccact acaaaaatat ga 1662

<210> 11
<211> 33
<212> DNA
<213> Artificial

<220>
<223> oligonucleotide as PCR primer

<400> 11
tcagatatcg atgacaaacc tgcaagatca aac 33

<210> 12
<211> 38
<212> DNA
<213> Artificial

<220>
<223> oligonucleotide as PCR primer

<400> 12
agaatgcggc cgcttacctc cttatagccc ggattatg 38

<210> 13
<211> 1362
<212> DNA
<213> Infectious bursal disease virus

<400> 13
atgacaaacc tgcaagatca aacccaacag attgttccgt tcatacggag ccttctgatg 60

ccaacaaccg gaccggcgtc cattccggac gacaccctgg agaagcacac tctcaggtca 120

gagacctcga cctacaattt gactgtgggg gacacagggt cagggctaat tgtctttttc 180

cctggattcc ctggctcaat tgtgggtgct cactacacac tgcagagcaa tgggaactac 240

aagttcgatc agatgctcct gactgcccag aacctaccgg ccagctacaa ctactgcaga 300

ctagtgagtc ggagtctcac agtgaggtca agcacactcc ctggtggcgt ttatgcacta 360

aacggcacca taaacgccgt gaccttccaa ggaagcctga gtgaactgac agatgttagc 420

tacaatgggt tgatgtctgc aacagccaac atcaacgaca aaattgggaa tgtcctggta 480

ggggaagggg tcactgtcct cagcctaccc acatcatatg atcttgggta tgtgaggctt 540

ggtgacccca ttcccgctat agggcttgac ccaaaaatgg tagctacatg cgacagcagt 600

gacaggccca gagtctacac cataactgca gccgatgatt accaattctc atcacagtac 660

caaccaggtg gggtaacaat cacactgttc tcagccaaca ttgatgctat cacaagcctc 720

agcattgggg gagagctcgt gtttcaaaca agcgtccaag gccttgtact gggcgccacc 780

atctacctta taggctttga tgggactgcg gtaatcacca gagctgtagc cgcagataat 840

gggctgacgg ccggcaccga caatcttatg ccattcaatc ttgtcattcc aaccaatgag 900

ataacccagc caatcacatc catcaaactg gagatagtga cctccaaaag tggtggtcag 960

gcaggggatc agatgtcatg gtcggcaagt gggagcctag cagtgacgat ccatggtggc 1020

aactatccag gggccctccg tcccgtcaca ctagtagcct acgaaagagt ggcaacagga 1080

tccgtcgtta cggtcgctgg ggtgagtaac ttcgagctga ttccaaatcc tgaactagca 1140

aagaacctgg ttacagaata cggccgattt gacccaggag ccatgaacta cacaaaattg 1200

atactgagtg agagggaccg tcttggcatc aagaccgtct ggccaacaag ggagtacact 1260

gattttcgtg agtacttcat ggaggtggcc gacctcaact ctcccctgaa gattgcagga 1320

gcatttggct tcaaagacat aatccgggct ataaggaggt aa 1362

<210> 14
<211> 32
<212> DNA
<213> Artificial

<220>
<223> oligonucleotide as PCR primer

<400> 14
acgcgtcgac atgttggtaa cacctctttt ac 32

<210> 15
<211> 35
<212> DNA
<213> Artificial

<220>
<223> oligonucleotide as PCR primer

<400> 15
ggaagatctt cattaacgtc taaaacgacg tgttc 35

<210> 16
<211> 1614
<212> DNA
<213> chicken infectious bronchitis virus

<400> 16
atgttggtaa cacctctttt actagtgact cttttgtgtg tactatgtag tgctgctttg 60

tatgacagta gttcttacgt ttactactac caaagtgcct ttagaccacc taatggttgg 120

catttacacg ggggtgctta tgcggtagtt aatatttcta gcgaatctaa taatgcaggc 180

tcttcacctg ggtgtattgt tggtactatt catggtggtc gtgttgttaa tgcttcttct 240

atagctatga cggcaccgtc atcaggtatg gcttggtcta gcagtcagtt ttgtactgca 300

cactgtaact tttcagatac tacagtgttt gttacacatt gttataaata tgatgggtgt 360

cctataactg gcatgcttca aaagaatttt ttacgtgttt ctgctatgaa aaatggccag 420

cttttctata atttaacagt tagtgtagct aagtacccta cttttaaatc atttcagtgt 480

gttaataatt taacatccgt atatttaaat ggtgatcttg tttacacctc taatgagacc 540

acagatgtta catctgcagg tgtttatttt aaagctggtg gacctataac ttataaagtt 600

atgagagaag ttaaagccct ggcttatttt gttaatggta ctgcacaaga tgttattttg 660

tgtgatggat cacctagagg cttgttagca tgccagtata atactggcaa tttttcagat 720

ggcttttatc cttttattaa tagtagttta gttaagcaga agtttattgt ctatcgtgaa 780

aatagtgtta atactacttt tacgttacac aatttcactt ttcataatga gactggcgcc 840

aaccctaatc ctagtggtgt tcagaatatt ctaacttacc aaacacaaac agctcagagt 900

ggttattata attttaattt ttcctttctg agtagttttg tttataagga gtctaatttt 960

atgtatggat cttatcaccc aagttgtaat tttagactag aaactattaa taatggcttg 1020

tggtttaatt cactttcagt ttcaattgct tacggtcctc ttcaaggtgg ttgcaagcaa 1080

tctgtcttta gtggtagagc aacttgttgt tatgcttatt catatggagg tccttcgctg 1140

tgtaaaggtg tttattcagg tgagttagct cttaattttg aatgtggact gttagtttat 1200

gttactaaga gcggtggctc tcgtatacaa acagccactg aaccgccagt tataactcga 1260

cacaattata ataatattac tttaaatact tgtgttgatt ataatatata tggcagaact 1320

ggccaaggtt ttattactaa tgtaaccgac tcagctgtta gttataatta tctagcagac 1380

gcaggtttgg ctattttaga tacatctggt tccatagaca tctttgttgt acaaggtgaa 1440

tatggtctta cttattataa ggttaaccct tgcgaagatg tcaaccagca gtttgtagtt 1500

tctggtggta aattagtagg tattcttact tcacgtaatg agactggttc tcagcttctt 1560

gagaaccagt tttacattaa aatcactaat ggaacacgtc gttttagacg ttaa 1614

<210> 17
<211> 39
<212> DNA
<213> Artificial

<220>
<223> oligonucleotide as PCR primer

<400> 17
ataagaatgc ggccgcatgt ccaacgagac aaattgtac 39

<210> 18
<211> 38
<212> DNA
<213> Artificial

<220>
<223> oligonucleotide as PCR primer

<400> 18
ataagaatgc ggccgcttta ggtgtaaaga ctactccc 38

<210> 19
<211> 678
<212> DNA
<213> chicken infectious bronchitis virus

<400> 19
atgtccaacg agacaaattg tactcttgac tttgaacagt cagttgagct ttttaaagag 60

tataatttat ttataactgc attcttgttg ttcttaacca taatacttca gtatggctat 120

gcaacaagaa gtaagtttat ttatatactg aaaatgatag tgttatggtg cttttggccc 180

cttaacattg cagtaggtgt aatttcatgt atatacccac caaacacagg aggtcttgtc 240

gcagcgataa tacttacagt gtttgcgtgt ctgtcttttg taggttattg gatccagagt 300

attagactct ttaagcggtg taggtcatgg tggtcattta acccagaatc taatgccgta 360

ggttcaatac tcctaactaa tggtcaacaa tgtaattttg ctatagagag tgtgccaatg 420

gtgctttctc caattataaa gaatggtgtt ctttattgtg agggtcagtg gcttgctaag 480

tgtgaaccag accacttgcc taaagatata tttgtttgta caccggatag acgtaatatc 540

taccgtatgg tgcagaaata tactggtgac caaagcggaa ataagaaacg gtttgctacg 600

tttgtctatg caaagcagtc agtagatact ggcgagctag aaagtgtagc aacaggaggg 660

agtagtcttt acacctaa 678

<210> 20
<211> 34
<212> DNA
<213> Artificial

<220>
<223> oligonucleotide as PCR primer

<400> 20
aaaactgcag tcatggcaag cggtaaggca actg 34


<210> 21
<211> 33
<212> DNA
<213> Artificial

<220>
<223> oligonucleotide as PCR primer

<400> 21
cgcggatcct caaagttcat tctctcctag ggc 33


<210> 22
<211> 1230
<212> DNA
<213> chicken infectious bronchitis virus

<400> 22
atggcaagcg gtaaggcaac tggaaagaca gacgccccag ctccagtcat caaactagga 60

ggaccaaagc cacctaaagt tggttcttct ggaaatgtat cttggtttca agcaataaaa 120

gccaagaagt taaattcacc tccgcctaag tttgaaggta gcggtgttcc tgataatgaa 180

aatctaaaac caagtcagca gcatggatat tggagacgcc aagctaggtt taagccaggt 240

aaaggtggaa gaaaaccagt cccagatgct tggtattttt actatactgg aacaggacca 300

gccgctaacc tgaattgggg tgatagccaa gatggtatag tgtgggttgc tggtaagggt 360

gctgatacta aatttagatc taatcagggt actcgtgact ctgacaagtt tgaccaatat 420

ccgctacggt tttcagacgg aggacctgat ggtaatttcc gttgggattt cattcctctg 480

aatcgtggca ggagtgggag atcaacagca gcttcatcag cggcatctag tagagcacca 540

tcacgtgaag tttcgcgtgg tcgcaggagt ggttctgaag atgatcttat tgctcgtgca 600

gcaaggataa ttcaggatca gcagaagaag ggttctcgca ttacaaaggc taaggctgat 660

gaaatggctc accgccggta ttgcaagcgc actattccac ctaattataa ggttgatcaa 720

gtgtttggtc cccgtactaa aggtaaggag ggaaattttg gtgatgacaa gatgaatgag 780

gaaggtatta aggatgggcg cgttacagca atgctcaacc tagttcctag cagccatgct 840

tgtcttttcg gaagtagagt gacgcccaga cttcaaccag atgggctgca cttgaaattt 900

gaatttacta ctgtggtccc acgtgatgat ccgcagtttg ataattatgt aaaaatttgt 960

gatcagtgtg ttgatggtgt aggaacacgt ccaacagatg atgaaccaag accaaagtca 1020

cgctcaagtt caaaacctgc aacaagagga aattctccag cgccaagaca gcagcgccct 1080

aagaaggaga aaaagccaaa gaagcaggat gatgaagtgg ataaagcatt gacctcagat 1140

gaggagagga acaatgcaca gctggaattt gatgatgaac ccaaggtaat taactggggg 1200

gattcagccc taggagagaa tgaactttga 1230


<210> 23
<211> 39
<212> DNA

<213> Artificial

<220>
<223> oligonucleotide as PCR primer

<400> 23
ttcttgcggc cgccatggca agacgagctc gcagaccga 39

<210> 24
<211> 38
<212> DNA
<213> Artificial

<220>
<223> oligonucleotide as PCR primer

<400> 24
ttcttgcggc cgctcagggc tgcgtccccc agtacatg 38

<210> 25
<211> 39
<212> DNA
<213> Artificial

<220>
<223> oligonucleotide as PCR primer

<400> 25
ttcttgcggc cgccatgcac gggaacggcg gacaaccgg 39

<210> 26
<211> 32
<212> DNA
<213> Artificial

<220>
<223> oligonucleotide as PCR primer

<400> 26
cgcggatcct cacactatac gtaccggggc gg 32

<210> 27
<211> 38
<212> DNA
<213> Artificial

<220>
<223> oligonucleotide as PCR primer

<400> 27
ttcttgcggc cgccatggct agcttgaaaa tgctgatc 38

<210> 28
<211> 36
<212> DNA
<213> Artificial

<220>
<223> oligonucleotide as PCR primer

<400> 28
ttcttgcggc cgcttattcg tcttcgcttt cttctg 36

<210> 29
<211> 33
<212> DNA
<213> Artificial

<220>
<223> oligonucleotide as PCR primer

<400> 29
ccggtcgaca tggaccgcca tttatttttg agg 33

<210> 30
<211> 33
<212> DNA
<213> Artificial

<220>
<223> oligonucleotide as PCR primer

<400> 30
ggaagatctt tacgatgctc caaaccagta gcc 33

<210> 31
<211> 54
<212> DNA
<213> Artificial

<220>
<223> oligonucleotide as PCR primer

<400> 31
tttgatatca tggaagccgt cattaaggca tttctgactg gataccctgg gaag 54

<210> 32
<211> 31
<212> DNA
<213> Artificial

<220>
<223> oligonucleotide as PCR primer

<400> 32
tttggatcct tatactattc tgctttcagg c 31

<210> 33
<211> 31
<212> DNA
<213> Artificial

<220>
<223> oligonucleotide as PCR primer

<400> 33
acgcgtcgac atggaagccg tcattaaggt g 31

<210> 34
<211> 32
<212> DNA
<213> Artificial

<220>
<223> oligonucleotide as PCR primer

<400> 34
tgctctagac tataaatttg tcaagcggag cc 32

<210> 35
<211> 32
<212> DNA
<213> Artificial

<220>
<223> oligonucleotide as PCR primer

<400> 35
aaactgcaga gatggggtca gagctctaca tc 32

<210> 36
<211> 31
<212> DNA
<213> Artificial

<220>
<223> oligonucleotide as PCR primer

<400> 36
cgaagatctt tattgactag tacagcacca c 31


<210> 37
<211> 33
<212> DNA
<213> Artificial

<220>
<223> oligonucleotide as PCR primer

<400> 37
aaactgcagc aatggccatc atttatctaa ttc 33


<210> 38
<211> 31
<212> DNA
<213> Artificial

<220>
<223> oligonucleotide as PCR primer

<400> 38
cgaagatctt catatgcaga ttctgcattg c 31


<210> 39
<211> 31
<212> DNA
<213> Artificial

<220>
<223> oligonucleotide as PCR primer

<400> 39
aaactgcaga tgaacactca aatcctgata c 31


<210> 40
<211> 31
<212> DNA
<213> Artificial

<220>
<223> oligonucleotide as PCR primer

<400> 40
tttggatcct tatatacaaa tagtgcaccg c 31


<210> 41
<211> 32
<212> DNA
<213> Artificial

<220>
<223> oligonucleotide as PCR primer

<400> 41
ccggtcgaca tggcgtctca aggcaccaaa cg 32


<210> 42
<211> 30
<212> DNA

<213>  Artificial

<220>
<223>  oligonucleotide as PCR primer

<400>  42
cgcggatcct taattgtcat actcctctgc                                30


<210>  43
<211>  35
<212>  DNA
<213>  Artificial

<220>
<223>  oligonucleotide as PCR primer

<400>  43
cgcgtcgaca tgaatccaaa tcagaaaata ataac                          35


<210>  44
<211>  31
<212>  DNA
<213>  Artificial

<220>
<223>  oligonucleotide as PCR primer

<400>  44
ggaagatctc tacttgtcaa tggtgaatgg c                              31

## Description

La présente invention a trait à une formule de vaccin permettant la vaccination des espèces aviaires, en particulier poulet. Elle a également trait à une méthode de vaccination correspondante.

On a déjà proposé par le passé des associations de vaccins contre un certain nombre de virus responsables de pathologies chez le poulet.

Les associations développées jusqu'à présent étaient réalisées à partir de vaccins inactivés ou de vaccins vivants. Leur mise en oeuvre pose des problèmes de compatibilité entre valences et de stabilité. Il faut en effet assurer à la fois la compatibilité entre les différentes valences de vaccin, que ce soit au plan des différents antigènes utilisés ou au plan des formulations elles-mêmes, il se pose également le problème de la conservation de tels vaccins combinés et de leur innocuité notamment en présence d'adjuvant. Ces vaccins sont en général assez coûteux.

Les demandes de brevet WO-A-90 11092, WO-A-93 19183, WO-A-94 21797 et WO-A-95 20660 ont fait usage de la technique récemment développée des vaccins polynucléotidiques. On sait que ces vaccins utilisent un plasmide apte à exprimer dans les cellules de l'hôte l'antigène inséré dans le plasmide. Toutes les voies d'administration ont été proposées (intrapéritonéale, intraveineuse, intramusculaire, transcutanée, intradermique, mucosale, etc.). Différents moyens de vaccination peuvent également être utilisés, tels que ADN déposé à la surface de particules d'or et projeté de façon à pénétrer dans la peau de l'animal (Tang et al,. Nature 356, 152-154, 1992) et les injecteurs par jet liquide permettant de transfecter à la fois dans la peau, le muscle, les tissus graisseux et les tissus mammaires (Furth et al., Analytical Biochemistry, 205, 365-368, 1992).

Les vaccins polynucléotidiques peuvent utiliser aussi bien des ADN nus que des ADN formulés par exemple au sein de liposomes ou de lipides cationiques.

L'invention se propose donc de fournir une formule de vaccin multivalent permettant d'assurer une vaccination contre un certain nombre de virus pathogènes aviaires.

Un autre objectif de l'invention est de fournir une telle formule de vaccin associant différentes valences tout en présentant tous les critères requis de compatibilité et de stabilité des valences entre elles.

Un autre objectif de l'invention est de fournir une telle formule de vaccin permettant d'associer différentes valences dans un même véhicule.

Un autre objectif de l'invention est de fournir un tel vaccin qui soit de mise en oeuvre aisée et peu coûteuse.

Un autre objectif, encore de l'invention est de fournir une méthode de vaccination des gallinacés qui permette d'obtenir une protection, y compris une protection multivalente, avec un niveau élevé d'efficacité et de longue durée, ainsi qu'une bonne innocuité et une absence de résidus

La présente invention a donc pour objet une formule de vaccin aviaire, comprenant au moins trois valences de vaccin polynucléotidique comprenant chacune un plasmide intégrant, de manière à l'exprimer in vivo dans les cellules hôtes, un gène d'une valence de pathogène aviaire, ces valences étant choisies parmi le groupe consistant en virus de la maladie de Marek (MDV), virus de la maladie de Newcastle (NDV), virus de la maladie de Gumboro (IBDV), virus de la bronchite infectieuse (IBV), virus de l'anémie infectieuse (CAV), virus de la laryngotrachéite infectieuse (ILTV), virus de l'encéphalomyélite (AEV ou encore virus de la leucose aviaire ALV), virus de la pneumovirose, et virus de la peste aviaire, les plasmides comprenant, pour chaque valence, un ou plusieurs des gènes choisis parmi le groupe consistant en gB et gD pour le virus de la maladie de Marek, HN et F pour le virus de la maladie de Newcastle, VP2 pour le virus de la maladie de Gumboro, S, M et N pour le virus de la bronchite infectieuse, C + NS1 pour le virus de l'anémie infectieuse, gB et gD pour le virus de la laryngotrachéite infectieuse, env et gag/pro pour le virus de l'encéphalomyélite, F et G pour le virus de la pneumovirose et HA, N et NP pour le virus de la peste aviaire.

Par valence, dans la présence invention, on entend au moins un antigène assurant une protection contre le virus du pathogène considéré, la valence pouvant contenir, à titre de sous-valence, un ou plusieurs gènes naturels ou modifiés d'une ou plusieurs souches du pathogène considéré.

Par gène d'agent pathogène, on entend non seulement le gène complet, mais aussi les séquences nucléotidiques différentes, y compris fragments, conservant la capacité à induire une réponse protectrice. La notion de gène recouvre les séquences nucléotidiques équivalentes à celles décrites précisément dans les exemples, c'est-à-dire les séquences différentes mais codant pour la même protéine. Elle recouvre aussi les séquences nucléotidiques d'autres souches du pathogène considéré, assurant une protection croisée ou une protection spécifique de souche ou de groupe de souche. Elle recouvre encore les séquences nucléotidiques qui ont été modifiées pour faciliter l'expression in vivo par l'animal hôte mais codant pour la même protéine.

De préférence, la formule de vaccin selon l'invention comprend trois valences choisies parmi Marek, Gumboro, Anémie infectieuse et Newcastle. On peut aussi y rajouter de préférence la valence bronchite infectieuse.

Sur cette base de 3, 4 ou 5 valences, on pourra ajouter une ou plusieurs des valences peste aviaire, laryngotrachéite, pneumovirose et encéphalomyélite.

La présente invention a également pour objet les élèments 1 à 14 suivants :
1. Formule de vaccin aviaire, comprenant au moins trois valences de vaccin polynucléotidique comprenant chacune un plasmide intégrant, de manière à l'exprimer in vivo dans les cellules hôtes, un gène d'une valence de pathogène aviaire, ces valences étant choisies parmi le groupe consistant en virus de la maladie de Marek, virus de la maladie de Newcastle, virus de la maladie de Gumboro, virus de l'anémie infectieuse, les plasmides comprenant, pour chaque valence, un ou plusieurs des gènes choisis parmi le groupe consistant en gB et gD pour le virus de la maladie de Marek, HN et F pour le virus de la maladie de Newcastle, VP2 pour le virus de la maladie de Gumboro, C + NS1 pour le virus de l'anémie infectieuse.
2. Formule de vaccin selon l'élément 1, caractérisée en ce que, pour la valence du virus de la maladie de Marek, elle comprend le gène gB seul.
3. Formule selon l'élément 1, caractérisée en ce qu'elle comprend les gènes HN et F du virus de la maladie de Newcastle, dans le même plasmide ou dans des plasmides différents.
4. Formule de vaccin selon l'élément 1, caractérisée en ce que le plasmide pour le virus de l'anémie infectieuse comprend C + NS1 dans le même plasmide.
5. Formule de vaccin selon l'un quelconque des éléments 1 à 4, caractérisée en ce qu'elle comporte en plus au moins une valence choisie parmi le groupe consistant en virus de la bronchite infectieuse, virus de la laryngotrachéite infectieuse, virus de l'encéphalomyélite, virus de la pneumovirose, et virus de la peste aviaire, les plasmides comprenant pour ces valences un ou plusieurs des gènes choisis parmi le groupe consistant en S, M et N pour le virus de la bronchite infectieuse, gB et gD pour le virus de la laryngotrachéite infectieuse, env et gag/pro pour le virus de l'encéphalomyélite, F et G pour le virus de la pneumovirose et HA, N et NP pour le virus de la peste aviaire.
6. Formule de vaccin selon l'élément 5, caractérisée en ce que, pour la valence virus de la bronchite infectieuse, elle comprend le gène S seul.
7. Formule de vaccin selon l'élément 5, caractérisée en ce que, pour la valence laryngotrachéite, la formule comprend le gène gB seul.
8. Formule de vaccin selon l'élément 5, caractérisée en ce que, pour la valence pneumovirose, la formule comprend les deux gènes F et G dans des plasmides différents ou dans un seul et même plasmide.
9. Formule de vaccin selon l'élément 5, caractérisée en ce que, pour la valence peste aviaire, la formule comprend le gène HA seul.
10. Formule de vaccin selon l'élément 5, caractérisée en ce que, pour la valence encéphalomyélite, la formule de vaccin comprend le gène env.
11. Formule de vaccin selon l'un quelconque des éléments 1 à 10, caractérisée en ce qu'il comprend de 10 ng à 1 mg, de préférence de 100 ng à 500 µg plus préférentiellement encore de 0,1 µg à 50 µg de chaque plasmide.
12. Utilisation d'un ou de plusieurs plasmides tels que décrits dans l'un quelconque des éléments 1 à 11, pour la fabrication d'un vaccin aviaire destiné à vacciner les animaux primo-vaccinés au moyen d'un premier vaccin choisi dans le groupe consistant en vaccin entier vivant, vaccin entier inactivé, vaccin de sous-unité, vaccin recombinant, ce premier vaccin présentant le ou les antigène(s) codé(s) par le ou les plasmide(s) ou antigène(s) assurant une protection croisée.
13. Kit de vaccination regroupant une formule de vaccin selon l'un quelconque des éléments 1 à 11 et un vaccin aviaire choisi dans le groupe consistant en vaccin entier vivant, vaccin entier inactivé, vaccin de sous-unité, vaccin recombinant, ce premier vaccin présentant l'antigène codé par le vaccin polynucléotidique ou un antigène assurant une protection croisée, pour une administration de ce dernier en primo-vaccination et pour un rappel avec la formule de vaccin.
14. Formule de vaccin selon l'un quelconque des éléments 1 à 11, accompagnée d'une notice indiquant que cette formule est utilisable en rappel d'un premier vaccin aviaire choisi dans le groupe consistant en vaccin entier vivant, vaccin entier inactivé, vaccin de sous-unité, vaccin recombinant, ce premier vaccin présentant l'Antigène codé par le vaccin polynucléotidique ou un antigène assurant une protection croisée.

En ce qui concerne la valence Marek, on peut mettre en oeuvre les deux gènes codant pour gB et gD, dans des plasmides différents ou dans un seul et même plasmide. On préfère toutefois n'utiliser que le gène gB.

Pour la valence Newcastle, on utilise de préférence les deux gènes HN et F intégrés dans deux plasmides différents ou dans un seul et même plasmide.

Pour la valence bronchite infectieuse, on préfère utiliser le gène S. Eventuellement, mais de façon moins préférée, on peut associer S et M dans un seul plasmide ou dans des

plasmides différents.

Pour la valence anémie infectieuse, on préfère associer les deux gènes C et NS1 dans le même plasmide.

Pour la valence laryngotrachéite infectieuse, on préfère utiliser le gène gB seul. Eventuellement, mais de façon moins préférée, on peut associer les deux gènes gB et gD dans des plasmides différents ou dans un seul et même plasmide.

Pour la valence pneumovirose, on préfère utiliser les deux gènes F et G, dans un seul plasmide ou dans des plasmides différents.

Pour la valence peste aviaire, on préfère utiliser le gène HA. Eventuellement, mais de façon moins préférée, on peut utiliser des associations HA et NP ou HA et N dans des plasmides différents ou dans un seul et même plasmide. De préférence, on associe dans le même vaccin les séquences HA de plus d'une souche de virus influenza, en particulier des différentes souches rencontrées sur le terrain. En revanche, NP assure une protection croisée et l'on pourra donc se contenter de la séquence d'une seule souche du virus.

Pour la valence encéphalomyélite, on préfère utiliser env.

La formule de vaccin selon l'invention pourra se présenter sous un volume de dose compris entre 0,1 et 1 ml et en particulier entre 0,3 et 0,5 ml.

La dose sera généralement comprise entre 10 ng et 1 mg, de préférence entre 100 ng et 500 µg et préférentiellement entre 0,1 µg et 50 µg par type de plasmide.

On utilisera de préférence des plasmides nus, simplement placés dans le véhicule de vaccination qui sera en général de l'eau physiologique ou analogue. On peut bien entendu utiliser toutes les formes de vaccin polynucléotidique décrites dans l'art antérieur et notamment formulés dans des liposomes.

Chaque plasmide comprend un promoteur apte à assurer l'expression du gène inséré sous sa dépendance dans les cellules hôtes. Il s'agira en général d'un promoteur eucaryote fort et en particulier d'un promoteur précoce du cytomégalovirus CMV-IE, d'origine humaine ou murine, ou encore éventuellement d'une autre origine telle que rat, cochon, cobaye.

De manière plus générale, le promoteur pourra être soit d'origine virale, soit d'origine cellulaire. Comme promoteur viral autre que CMV-IE, on peut citer le promoteur précoce ou tardif du virus SV 40 ou le promoteur LTR du virus du Sarcome de Rous. Il peut aussi s'agir d'un promoteur du virus dont provient le gène, par exemple le promoteur propre au gène.

Comme promoteur cellulaire, on peut citer le promoteur d'un gène du cytosquelette, tel que par exemple le promoteur de la desmine (Bolmont et al., Journal of Submicroscopic Cytology and Pathology, 1990, 22, 117-122 ; et ZHENLIN et al., Gene, 1989, 78, 243-254), ou encore le promoteur de l'actine.

Lorsque plusieurs gènes sont présents dans le même plasmide, ceux-ci peuvent être présentés dans la même unité de transcription ou dans deux unités différentes.

La combinaison des différentes valences du vaccin selon l'invention peut être effectuée, de préférence, par mélange de plasmides polynucléotidiques exprimant le ou les antigènes de chaque valence, mais on peut également prévoir de faire exprimer des antigènes de plusieurs valences par un même plasmide.

L'invention a encore pour objet des formules de vaccin monovalent comprenant un ou plusieurs plasmides codant pour un ou plusieurs gènes de l'un des virus ci-dessus, les gènes étant ceux décrits plus haut. En dehors de leur caractère monovalent, ces formules peuvent reprendre les caractéristiques énoncées plus haut en ce qui concerne le choix des gènes, leurs combinaisons, la composition des plasmides, les volumes de dose, les doses, etc.

Les formules de vaccin monovalent peuvent aussi être utilisées (i) pour la préparation d'une formule de vaccin polyvalent tel que décrit plus haut, (ii) à titre individuel contre la pathologie propre, (iii) associées à un vaccin d'un autre type (entier vivant ou inactivé, recombinant, sous-unité) contre une autre pathologie, ou (iv) comme rappel d'un vaccin comme décrit ci-après.

La présente invention a en effet encore pour objet l'utilisation d'un ou de plusieurs plasmides selon l'invention pour la fabrication d'un vaccin aviaire destiné à vacciner des animaux primo-vaccinés au moyen d'un premier vaccin classique (monovalent ou multi-valent), du type de ceux de l'art antérieur, choisi notamment dans le groupe consistant en vaccin entier vivant, vaccin entier inactivé, vaccin de sous-unité, vaccin recombinant, ce premier vaccin présentant (c'est-à-dire contenant ou pouvant exprimer) le ou les antigène(s) codé(s) par le ou les plasmides ou antigène(s) assurant une protection croisée.

De manière remarquable, le vaccin polynucléotidique a un effet de rappel puissant se traduisant par une amplification de la réponse immunitaire et l'instauration d'une immunité de longue durée.

De manière générale, les vaccins de primo-vaccination pourront être choisis parmi les vaccins commerciaux disponibles auprès des différents producteurs de vaccins vétérinaires.

L'invention a aussi pour objet un kit de vaccination regroupant une formule de vaccin selon l'invention et un vaccin de primo-vaccination tel que décrit ci-dessus. Elle a aussi trait à une formule de vaccin selon l'invention accompagnée d'une notice indiquant l'usage de cette formule comme rappel d'une primo-vaccination telle que décrite ci-avant.

La présente invention a également pour objet une méthode de vaccination aviaire, comprenant l'administration d'une formule de vaccin efficace telle que décrit plus haut. Cette méthode de vaccination comprend l'administration d'une ou de plusieurs doses de formule de vaccin, ces doses pouvant être administrées successivement dans un court laps de temps et/ou successivement à des moments éloignés l'un de l'autre.

Les formules de vaccin selon l'invention pourront être administrées, dans le cadre de cette méthode de vaccination, par les différentes voies d'administration proposées dans l'art antérieur dans le cadre de la vaccination polynucléotidique et au moyen des techniques d'administration connues.

On visera en particulier les voies intramusculaire, in ovo, intra-oculaire, la nébulisation et l'eau de boisson.

L'efficacité de la présentation des antigènes au système immunitaire varie en fonction des tissus. En particulier, les muqueuses de l'arbre respiratoire servent de barrière à l'entrée des pathogènes et sont associées à des tissus lymphoides qui supportent une immunité locale. En outre, l'administration d'un vaccin par contact avec les muqueuses, en particulier muqueuse buccale, pharyngée et région bronchique présente un intérêt certain pour la vaccination de masse.

En conséquence, les voies d'administration mucosales font partie d'un mode d'administration préféré pour l'invention, utilisant notamment la nébulisation ou spray ou l'eau de boisson. Les formules de vaccin et méthodes de vaccination selon l'invention pourront être appliquées dans ce cadre.

L'invention a encore pour objet la méthode de vaccination consistant à faire une primo-vaccination telle que décrite ci-dessus et un rappel avec une formule de vaccin selon l'invention.

Dans une forme de mise en oeuvre préférée du procédé selon l'invention, on administre dans un premier temps, à l'animal, une dose efficace du vaccin de type classique, notamment inactivé, vivant, atténué ou recombinant, ou encore un vaccin de sous-unité de façon à assurer une primo-vaccination, et, après un délai de préférence de 2 à 6 semaines, on assure l'administration du vaccin polyvalent ou monovalent selon l'invention.

L'invention concerne aussi la méthode de préparation des formules de vaccin, à savoir la préparation des valences ec leurs mélanges, telle qu'elle ressort de cette description.

L'invention va être maintenant décrite plus en détails à l'aide de modes de réalisation de l'invention pris en référence aux dessins annexés.

### Liste des figures

- Figure N° 1 :: Plasmide pVR1012
- Figure N° 2 :: Plasmide pAB045
- Figure N° 3 :: Plasmide pAB080
- Figure N° 4 :: Séquence du gène NDV HN souche Texas GB
- Figure N° 5 :: Plasmide pAB046
- Figure N° 6 :: Séquence du gène NDV F souche Texas GB
- Figure N° 7 :: Plasmide pAB047
- Figure N° 8 :: Séquence du gène IBDV VP2 souche Faragher
- Figure N° 9 :: Plasmide pAB048
- Figure N° 10 :: Séquence du gène IBV S souche Massachusetts 41
- Figure N° 11 :: Plasmide pAB049
- Figure N° 12 :: Séquence du gène IBV M souche Massachusetts 41
- Figure N° 13 :: Plasmide pAB050
- Figure N° 14 :: Séquence du gène IBV N souche Massachusetts 41
- Figure N° 15 :: Plasmide pAB051
- Figure N° 16 :: Plasmide pAB054
- Figure N° 17 :: Plasmide pAB055
- Figure N° 18 :: Plasmide pAB076
- Figure N° 19 :: Plasmide pAB089
- Figure N° 20 :: Plasmide pAB086
- Figure N° 21 :: Plasmide pAB081
- Figure N° 22 :: Plasmide pAB082
- Figure N° 23 :: Plasmide pAB077
- Figure N° 24 :: Plasmide pAB078
- Figure N° 25 :: Plasmide pAB088
- Figure N° 26 :: Plasmide pAB079

### Liste des séquences SEQ ID N°

- SEQ ID N° 1 :: Oligonucléotide AB062
- SEQ ID N° 2 :: Oligonucléotide AB063
- SEQ ID N° 3 :: Oligonucléotide AB148
- SEQ ID N° 4 :: Oligonucléotide AB149
- SEQ ID N° 5 :: Oligonucléotide AB072
- SEQ ID N° 6 :: Oligonucléotide AB073
- SEQ ID N° 7 :: Séquence du gène NDV HN souche Texas GB
- SEQ ID N° 8 :: Oligonucléotide AB091
- SEQ ID N° 9 :: Oligonucléotide AB092
- SEQ ID N° 10 :: Séquence du gène NDV F souche Texas GB
- SEQ ID N° 11 :: Oligonucléotide AB093
- SEQ ID N° 12 :: Oligonucléotide AB094
- SEQ ID N° 13 :: Séquence du "gène" IBDV VP2 souche Faragher
- SEQ ID N° 14 :: Oligonucléotide AB095
- SEQ ID N° 15 :: Oligonucléotide AB096
- SEQ ID N° 16 :: Séquence du gène IBV S souche Massachusetts 41
- SEQ ID N° 17 :: Oligonucléotide AB097
- SEQ ID N° 18 :: Oligonucléotide AB098
- SEQ ID N° 19 :: Séquence du gène IBV M souche Massachusetts 41
- SEQ ID N° 20 :: Oligonucléotide AB099
- SEQ ID N° 21 :: Oligonucléotide AB100
- SEQ ID N° 22 :: Séquence du gène IBV N souche Massachusetts 41
- SEQ ID N° 23 :: Oligonucléotide CD064
- SEQ ID N° 24 :: Oligonucléotide CD065
- SEQ ID N° 25 :: Oligonucléotide CD066
- SEQ ID N° 26 :: Oligonucléotide AB105
- SEQ ID N° 27 :: Oligonucléotide AB140
- SEQ ID N° 28 :: Oligonucléotide AB141
- SEQ ID N° 29 :: Oligonucléotide AB164
- SEQ ID N° 30 :: Oligonucléotide AB165
- SEQ ID N° 31 :: Oligonucléotide AB160
- SEQ ID N° 32 :: Oligonucléotide AB161
- SEQ ID N° 33 :: Oligonucléotide AB150
- SEQ ID N° 34 :: Oligonucléotide AB151
- SEQ ID N° 35 :: Oligonucléotide AB152
- SEQ ID N° 36 :: Oligonucléotide AB153
- SEQ ID N° 37 :: Oligonucléotide AB142
- SEQ ID N° 38 :: Oligonucléotide AB143
- SEQ ID N° 39 :: Oligonucléotide AB144
- SEQ ID N° 40 :: Oligonucléotide AB145
- SEQ ID N° 41 :: Oligonucléotide AB156
- SEQ ID N° 42 :: Oligonucléotide AB158
- SEO ID N° 43 :: Oligonucléotide AB146
- SEQ ID N° 44 :: Oligonucléotide AB147

### EXEMPLES

### Exemple 1 : Culture des virus

Les virus sont cultivés sur le système cellulaire approprié jusqu'à obtention d'un effet cytopathique. Les systèmes cellulaires à utiliser pour chaque virus sont bien connus de l'homme du métier. Brièvement, des cellules sensibles au virus utilisé, cultivées en milieu minimum essentiel de Eagle (milieu "MEM) ou un autre milieu approprié, sont inoculées avec la souche virale étudiée en utilisant une multiplicité d'infection de 1. Les cellules infectées sont alors incubées à 37°C pendant le temps nécessaire à l'apparition d'un effet cytopathique complet (en moyenne 36 heures).

### Exemple 2 : Extraction des ADNs génomiques viraux

Après culture, le surnageant et les cellules lysées sont récoltées et la totalité de la suspension virale est centrifugée à 1000 g pendant 10 minutes à + 4°C pour éliminer les débris cellulaires. Les particules virales sont alors récoltées par ultracentrifugation à 400000 g pendant 1 heure à + 4°C. Le culot est repris dans un volume minimum de tampon (Tris 10 mM, EDTA 1 mM). Cette suspension virale concentrée est traitée par la protéinase K (100 µg/ml final) en présence de sodium dodecyl sulfate (SDS) (0,5% final) pendant 2 heures à 37°C. L'ADN viral est ensuite extrait avec un mélange de phénol/chloroforme, puis précipité avec 2 volumes d'éthanol absolu. Après une nuit à - 20°C, l'ADN est centrifugé à 10000 g pendant 15 minutes à + 4°C. Le culot d'ADN est séché, puis repris dans un volume minimum d'eau ultrapure stérile. Il peut alors être digéré par des enzymes de restriction.

### Exemple 3 : Isolement des ARNs génomiques viraux

Les virus à ARN ont été purifiés selon les techniques bien connues de l'homme du métier. L'ARN viral génomique de chaque virus a été ensuite isolé en utilisant la technique d'extraction "thiocyanate de guanidium/phénol-chloroforme" décrite par P. Chomczynski et N. Sacchi (Anal. Biochem. 1987. 162. 156-159).

### Exemple 4 : Techniques de biologie moléculaire

Toutes les constructions de plasmides ont été réalisées en utilisant les techniques standards de biologie moléculaire décrites par J. Sambrook *et al. (Molecular Cloning: A Laboratory Manual.* 2^{nd} Edition. Cold Spring Harbor Laboratory. Cold Spring Harbor. New York. 1989). Tous les fragments de restriction utilisés pour la présente invention ont été isolés en utilisant le kit "Genectean" (BIO101 Inc. La Jolla, CA).

### Exemple 5 : Technique de RT-PCR

Des oligonucléotides spécifiques (comportant à leurs extrémités 5' des sites de restriction pour faciliter le clonage des fragments amplifiés) ont été synthétisés de telle façon qu'ils couvrent entièrement les régions codantes des gènes devant être amplifiés (voir exemples spécifiques). La réaction de transcription inverse (RT) et l'amplification en chaîne par polymérase (PCR) ont été effectuées selon les techniques standards (Sambrook J. *et al.* 1989). Chaque réaction de RT-PCR a été faite avec un couple d'amplimers spécifiques et en prenant comme matrice l'ARN génomique viral extrait. L'ADN complémentaire amplifié a été extrait au phénol/chloroforme/alcoolisoamytique (25:24:1) avant d'être digéré par les enzymes de restriction.

### Exemple 6 : plasmide pVR1012

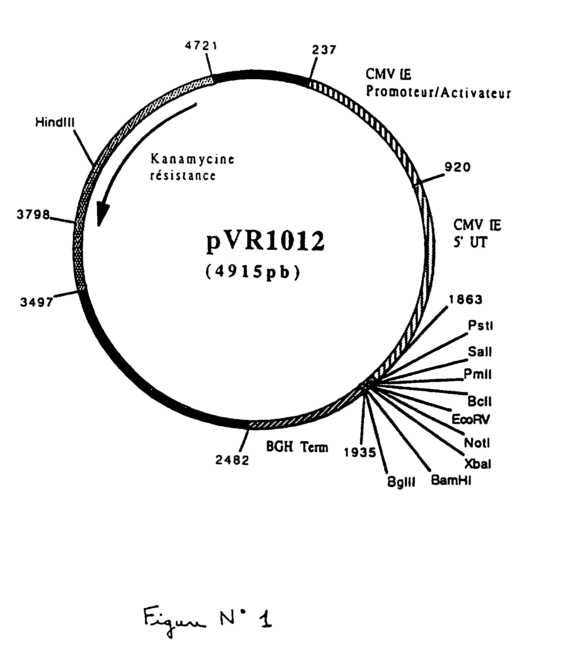

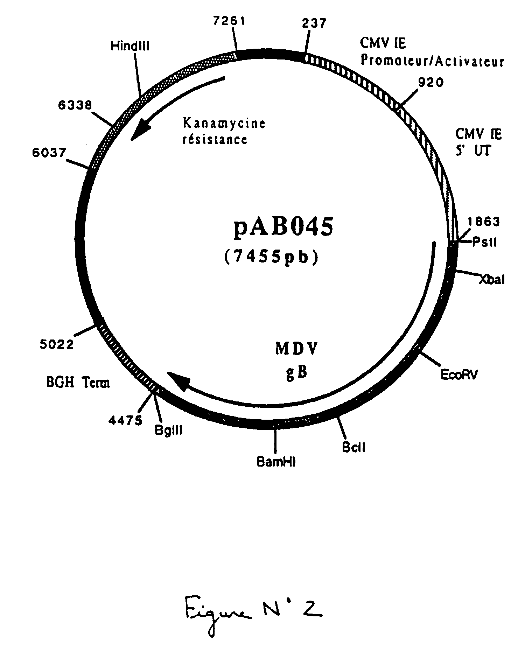

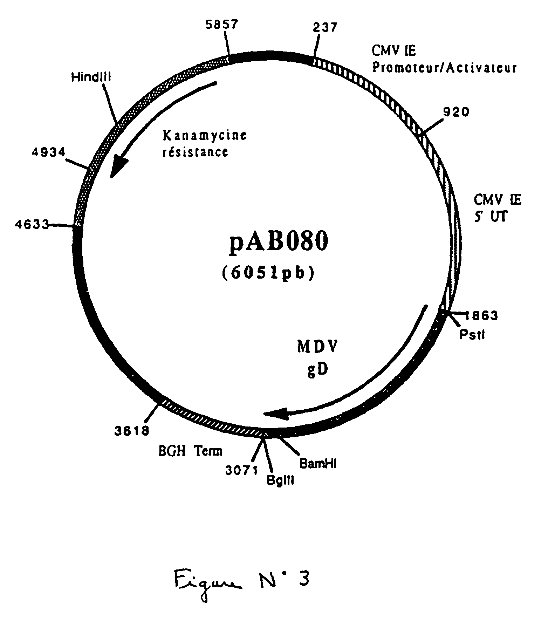

Le plasmide pVR1012 (Figure N° 1) a été obtenu auprès de Vical Inc. San Diego, CA, USA; Sa construction a été décrite dans J. Hartikka *et al.* (Human Gene Therapy. 1996. 7. 1205-1217).

### Exemple 7 : Construction du plasmide pAB045 (gène MDV g8)

Une réaction de PCR a été réalisée avec l'ADN génomique du virus de la maladie de Marek (MDV) (Souche RB1B) (L. Ross *et al.* J. Gen. Virol. 1989. 70. 1789-1804), préparé selon la technique de l'exemple 2, et avec les oligonucléotides suivants: 
AB062 (37 mer) (SEQ ID N° 1)
5'AAAACTGCAGACTATGCACTATTTTAGGCGGAATTGC 3'
 
AB063 (35 mer) (SEQ ID N° 2)
5'GGAAGATCTTTACACAGCATCATCTTCTGAGTCTG 3'
 pour isoler le gène codant pour la glycoprotéine gB du virus MDV sous la forme d'un fragment Pstl-BgIII. Après purification, le produit de PCR de 2613 pb a été digéré par *Pst*I et *Bgl*II pour isoler un fragment PstI-BglII de 2602 pb. Ce fragment a été ligaturé avec le vecteur pVR1012 (exemple 6), préalablement digéré avec *Pst*I et *Bgl*II, pour donner le plasmide pAB045 (7455 pb) (Figure N ° 2).

### Exemple 8 : Construction du plasmide pAB080 (gène MDV gD)

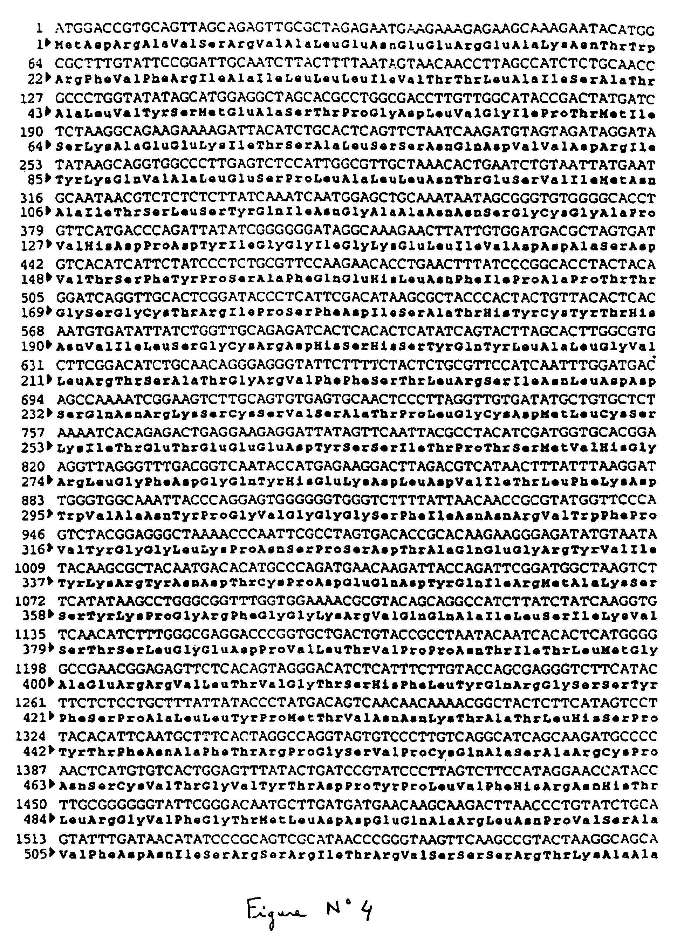

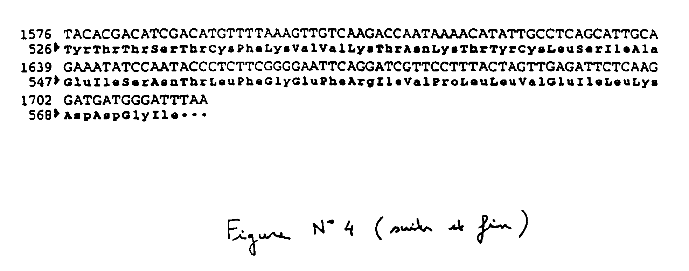

Une réaction de PCR a été réalisée avec l'ADN génomique du virus de la maladie de Marek (MDV) (Souche R818) (L. Ross *et al.* J. Gen. Virol. 1991. 72. 949-954), préparé selon la technique de l'exemple 2, et avec les oligonucléotides suivants: 
AB148 (29 mer) (SEQ ID N° 3)
5'AAACTGCAGATGAAAGTATTTTTTTTTAG 3'
 
AB149 (32 mer) (SEQ ID N° 4)
5'GGAAGATCTTTATAGGCGGGAATATGCCCGTC 3'
 pour isoler le gène codant pour la glycoprotéine gD du virus MDV sous la forme d'un fragment Pstl-BgIII. Après purification, le produit de PCR de 1215 pb a été digéré par *Pst*I et *Bgl*II pour isoler un fragment Pstl-BgIII de 1199 pb. Ce fragment a été ligaturé avec le vecteur pVR1012 (exemple 6), préalablement digéré avec *Pst*I et *Bgl*II, pour donner le plasmide pAB080 (6051 pb) (Figure N° 3).

### Exemple 9 : Construction du plasmide pAB046 (gène NDV HN)

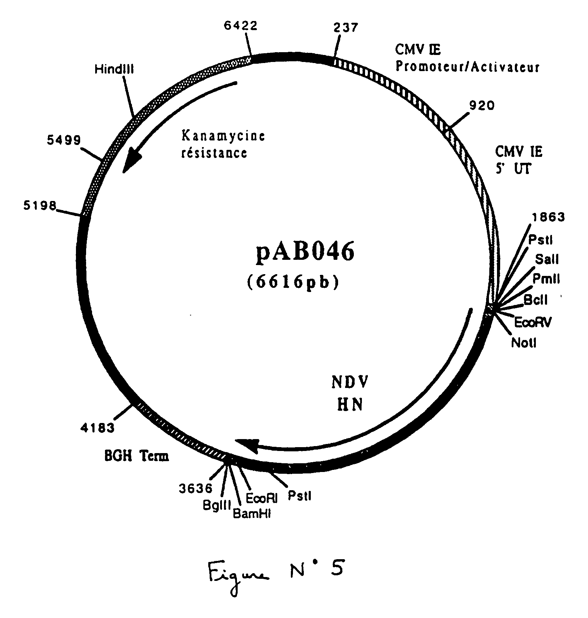

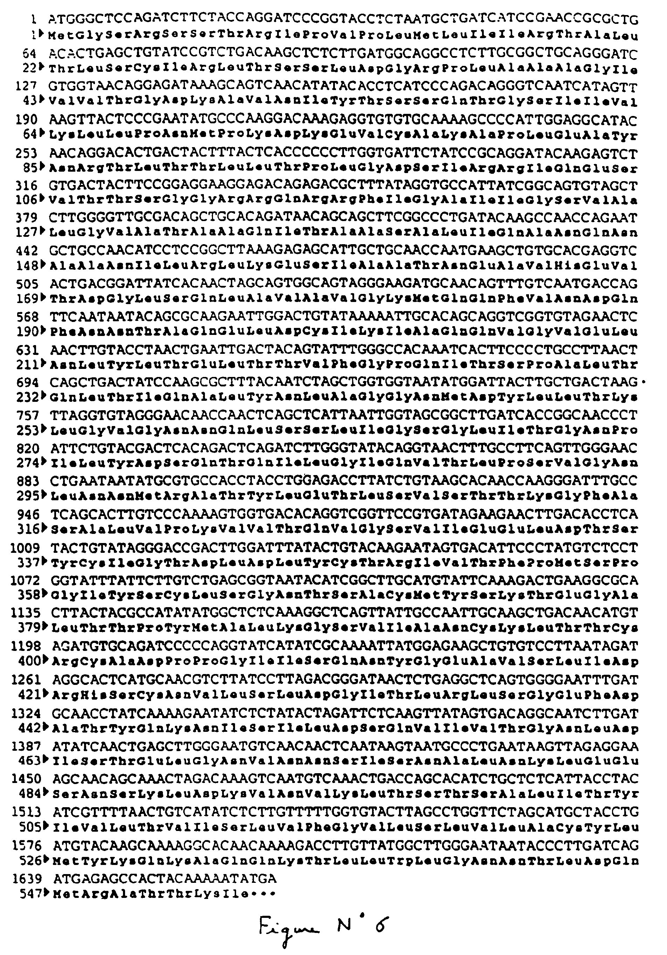

Une réaction de RT-PCR selon la technique de l'exemple 5 a été réalisée avec l'ARN génomique du virus de la maladie de Newcastle (NDV) (Souche Texas GB), préparé selon la technique de l'exemple 3, et avec les oligonucléotides suivants: 
AB072 (39 mer) (SEQ ID N° 5)
5'ATAAGAATGCGGCCGCCATGGACCGTGCAGTTAGCAGAG 3'
 
AB073 (34 mer) (SEQ ID N° 6)
5'CGCGGATCCTTAAATCCCATCATCCTTGAGAATC 3'
 pour isoler le gène codant pour la glycoprotéine HN du virus NDV souche Texas GB (Figure N° 4 et SEQ ID N° 7) sous la forme d'un fragment NotI-BamHI. Après purification, le produit de RT-PCR de 1741 pb a été digéré par *Not*I et *Bam*HI pour isoler un fragment Notl-BamHI de 1723 pb. Ce fragment a été ligaturé avec le vecteur pVR1012 (exemple 6), préalablement digéré avec *Not*I et *Bam*HI, pour donner le plasmide pAB046 (6616 pbl (Figure N° 5).

### Exemple 10 : Construction du plasmide pAB047 (gène NDV F)

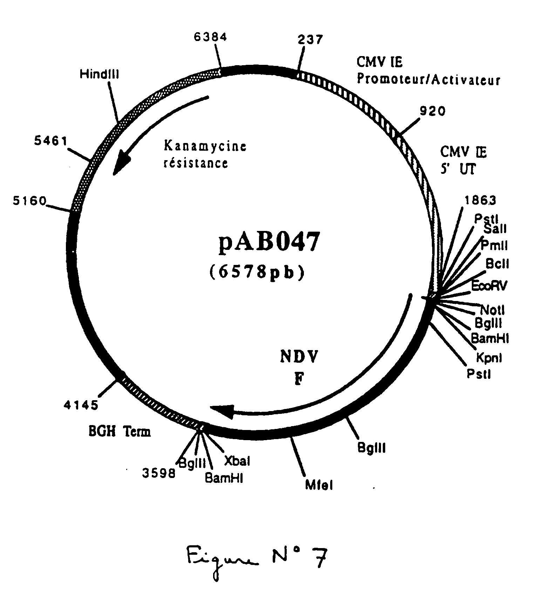

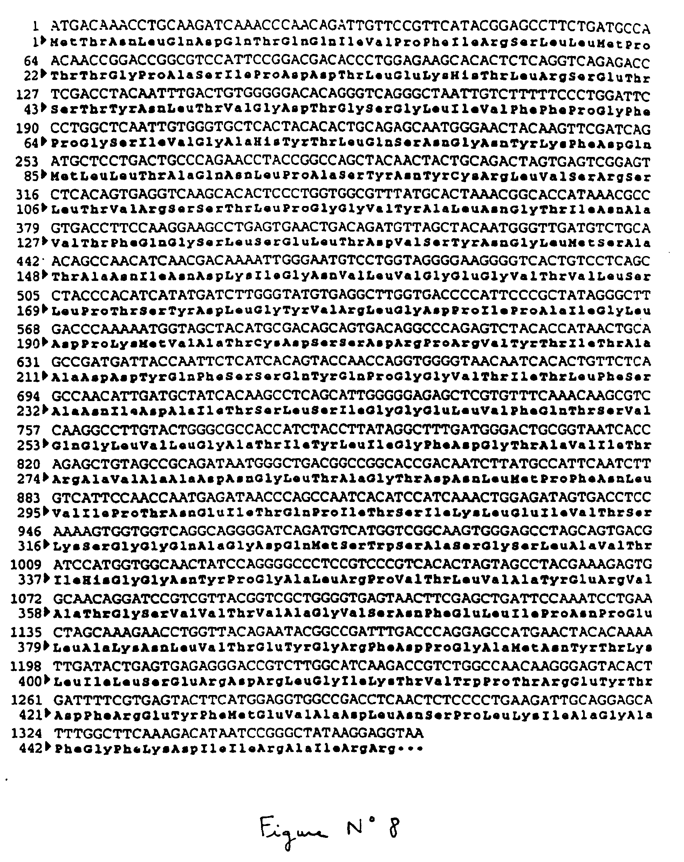

Une réaction de RT-PCR selon la technique de l'exemple 5 a été réalisée avec l'ARN génomique du virus de la maladie de Newcastle (NDV) (Souche Texas GB), préparé selon la technique de l'exemple 3, et avec les oligonucléotides suivants: 
AB091 (37 mer) (SEQ ID N° 8)
5'AGAATGCGGCCGCGATGGGCTCCAGATCTTCTACCAG 3'
 
AB092 (34 mer) (SEQ ID N° 9)
5'TGCTCTAGATCATATTTTTGTAGTGGCTCTCATC 3'
 pour isoler le gène codant pour la glycoprotéine F du virus NDV souche Texas GB (Figure N° 6 et SEQ ID N° 10) sous la forme d'un fragment Notl-Xbal. Après purification le produit de RT-PCR de 1684 pb a été digéré par *Not*I et *Xba*I pour isoler un fragment Notl-Xbal de 1669 pb. Ce fragment a été ligaturé avec le vecteur pVR1012 (exemple 6), préalablement digéré avec *Not*I et *Xba*I*,* pour donner le plasmide pAB047 (6578 pb) (Figure N° 7).

### Exemple 11 : Construction du plasmide pAB048 (gène IBDV VP2)

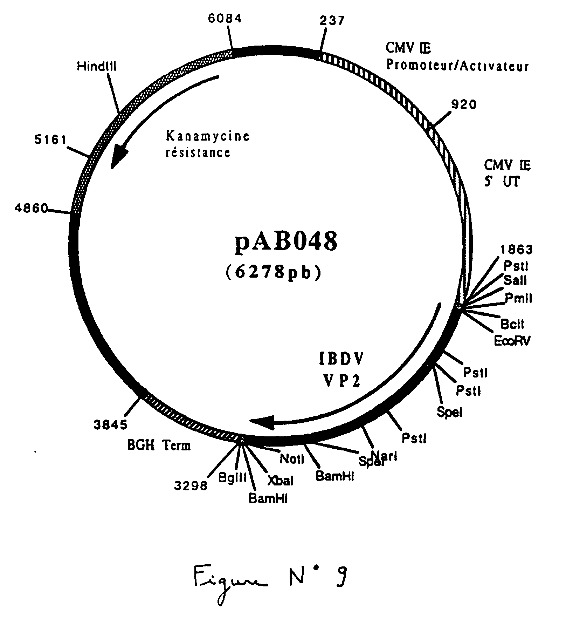

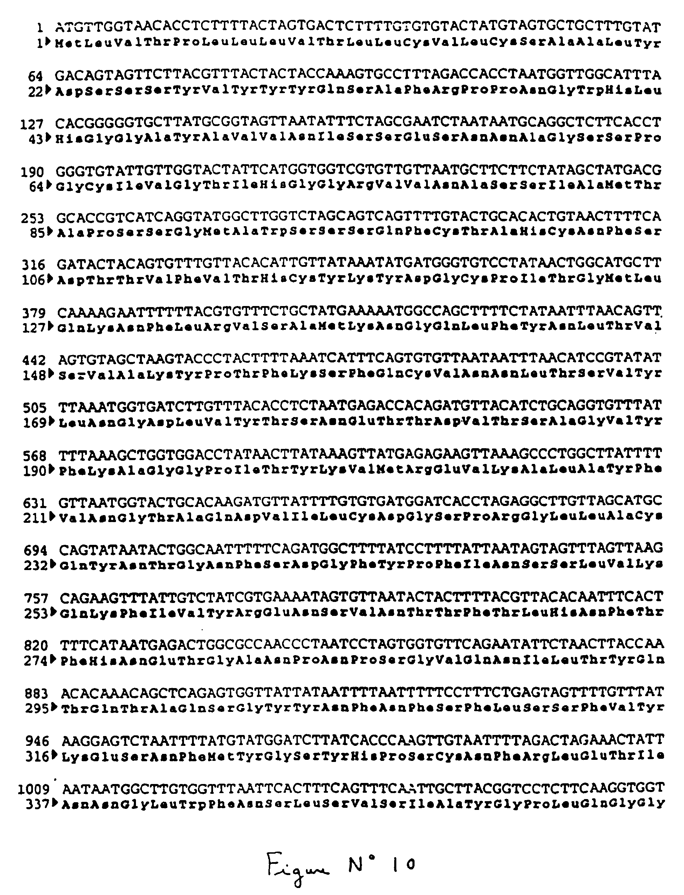

Une réaction de RT-PCR selon la technique de l'exemple 5 a été réalisée avec l'ARN génomique du virus de la maladie de Gumboro (IBDV) (Souche Faragher), préparé selon a technique de l'exemple 3, et avec les oligonucléotides suivants: 
AB093 (33 mer) (SEQ ID N° 11)
5'TCAGATATCGATGACAAACCTGCAAGATCAAAC 3'
 AB094 (38 mer) (SEQ ID N° 12) 5'AGAATGCGGCCGCTTACCTCCTTATAGCCCGGATTATG 3' pour isoler la séquence codant pour la protéine VP2 du virus IBDV souche faragher (Figure N° 8 et SEQ ID N° 13) sous la forme d'un fragment EcoRV-Notl. Après purification, le produit de RT-PCR de 1384 pb a été digéré par *Eco*RV et *Not*I pour isoler un fragment EcoRV-Notl de 1367 pb. Ce fragment a été ligaturé avec le vecteur pVR1012 (exemple 6), préalablement digéré avec *Eco*RV et *Not*I*,* pour donner le plasmide pAB048 (6278 pb) (Figure N° 9).

### Exemple 12 : Construction du plasmide pAB049 (gène IBV S1)

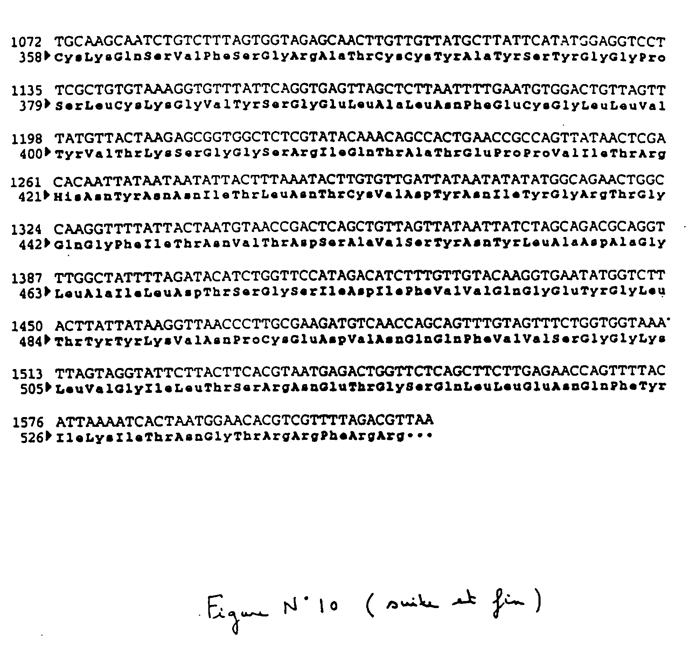

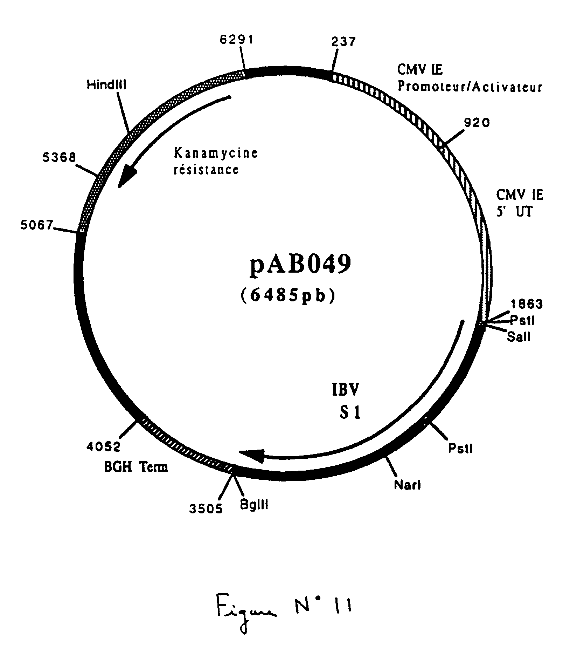

Une réaction de RT-PCR selon la technique de l'exemple 5 a été réalisée avec l'ARN génomique du virus de la bronchite infectieuse du poulet (IBV) (Souche Massachusetts 41), préparé selon a technique de l'exemple 3, et avec les oligonucléotides suivants: 
AB095 (32 mer) (SEQ ID N° 14)
5'ACGCGTCGACATGTTGGTAACACCTCTTTTAC 3'
 
AB096 (35 mer) (SEQ ID N° 15)
5'GGAAGATCTTCATTAACGTCTAAAACGACGTGTTC 3'
 pour isoler la séquence codant pour la sous-unité S1 de la glycoprotéine S du virus IBV souche Massachusetts 41 (Figure N° 10 et SEQ ID N° 16) sous la forme d'un fragment Sall-BgIII. Après purification, le produit de RT-PCR de 1635 pb a été digéré par *Sal*I et *Bgl*II pour isoler un fragment Sall-BgIII de 1622 pb. Ce fragment a été ligaturé avec le vecteur pVR1012 (exemple 6), préalablement digéré avec *Sal*I et *Bgl*II, pour donner le plasmide pAB049 (6485 pb) (Figure N° 11).

### Exemple 13 : Construction du plasmide pAB050 (gène IBV M)

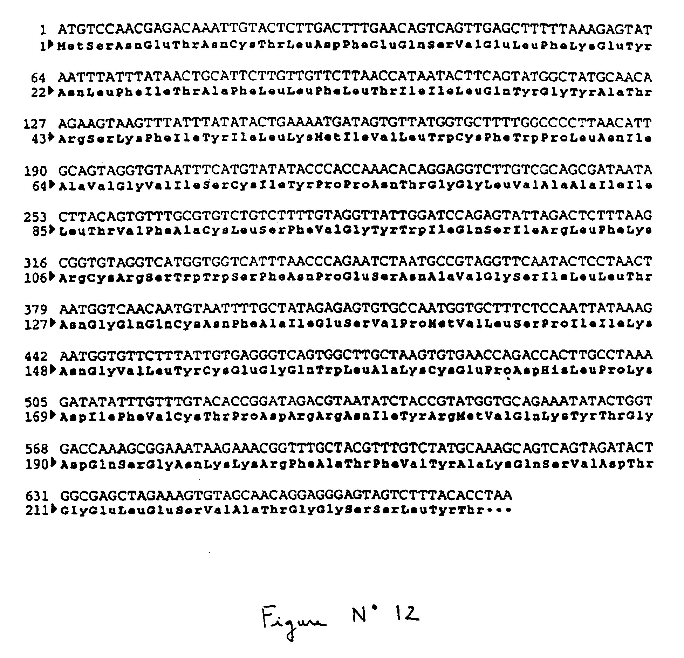

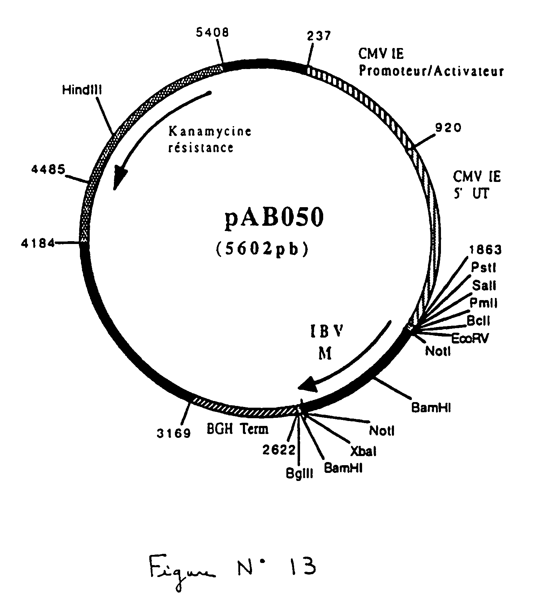

Une réaction de RT-PCR selon la technique de l'exemple 5 a été réalisée avec l'ARN génomique du virus de la bronchite infectieuse du poulet (IBV) (Souche Massachusetts 41), préparé selon la technique de l'exemple 3, et avec les oligonucléotides suivants: 
AB097 (37 mer) (SEQ ID N° 17)
5'ATAAGAATGCGGCCGCATGTCCAACGAGACAAATTGTAC 3'
 
AB098 (38 mer) (SEQ ID N° 18)
5'ATAAGAATGCGGCCGCTTTAGGTGTAAAGACTACTCCC 3'
 pour isoler le gène codant pour la glycoprotéine M du virus IBV souche Massachusetts 41 (Figure N° 12 et SEQ ID N° 19) sous la forme d'un fragment NotI-NotI. Après purification, le produit de RT-PCR de 710 pb a été digéré par *Not*I pour isoler un fragment Notl-Notl de 686 pb. Ce fragment a été ligaturé avec le vecteur pVR1012 (exemple 6), préalablement digéré avec *Not*I, pour donner le plasmide pAB050 (5602 pb) qui contient le gène IBV M dans la bonne orientation par rapport au promoteur (Figure N° 13).

### Exemple 14 : Construction du plasmide pAB051 (gène IBV N)

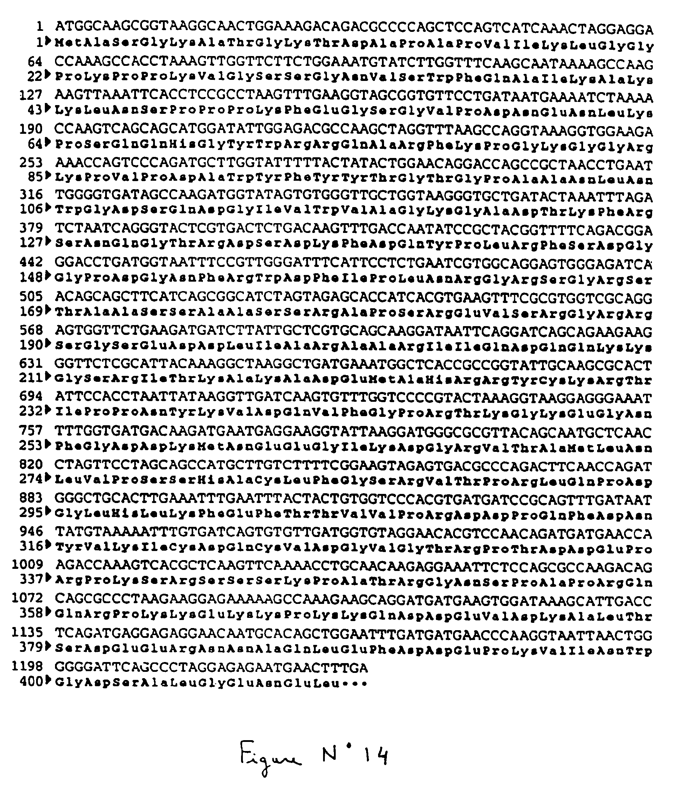

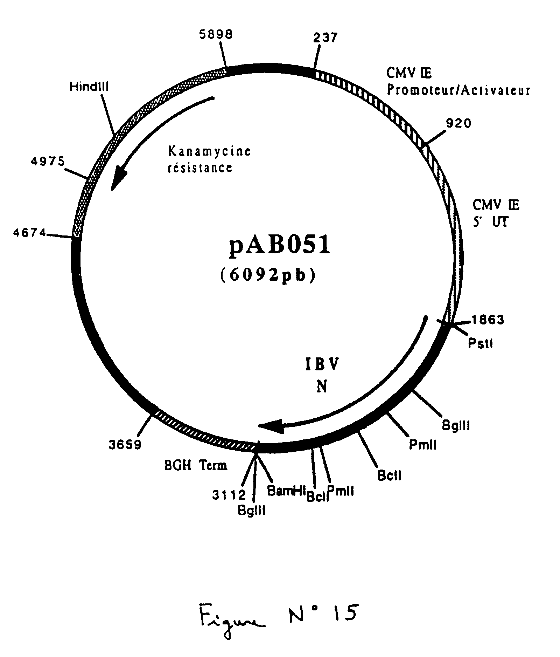

Une réaction de RT-PCR selon la technique de l'exemple 5 a été réalisée avec l'ARN génomique du virus de la bronchite infectieuse du poulet (IBV) (Souche Massachusetts 41), préparé selon la technique de l'exemple 3, et avec les otigonuctéotides suivants: 
AB099 (34 mer) (SEQ ID N° 20)
5'AAAACTGCAGTCATGGCAAGCGGTAAGGCAACTG 3'
 
AB100 (33 mer) (SEQ ID N° 21)
5'CGCGGATCCTCAAAGTTCATTCTCTCCTAGGGC 3'
 pour isoler le gène codant pour la protéine N du virus IBV souche Massachusetts 41 (Figure N° 14 et SEQ ID N° 22) sous la forme d'un fragment PstI-BamHI. Après purification, le produit de RT-PCR de 1250 pb a été digéré par *Pst*I et *Bam*HI pour isoler un fragment PstI-BamHI de 1233 pb. Ce fragment a été ligaturé avec le vecteur pVR1012 (exemple 6), préalablement digéré avec *Pst*I et *Bam*HI*,* pour donner le plasmide pAB051 (6092 pb) (Figure N° 15).

### Exemple 15 : Construction du plasmide pAB054 (gène CAV VP1)

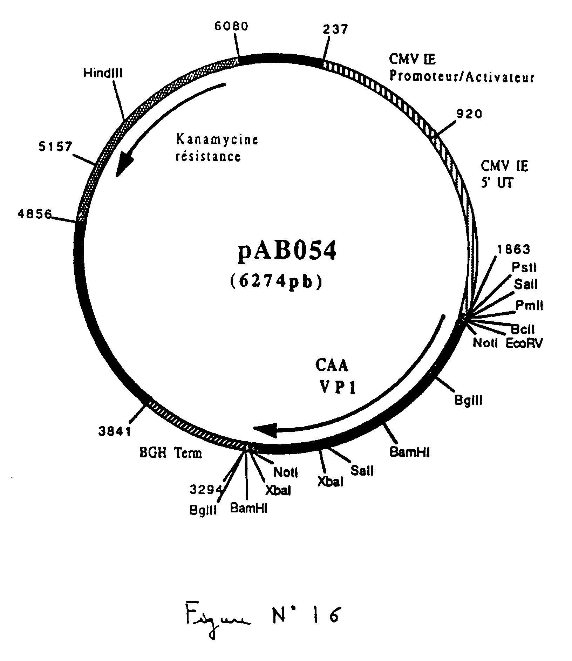

Une réaction de PCR a été réalisée avec l'ADN génomique du virus de l'anémie du poulet (CAV) (Souche Cuxhaven-1) (B. Meehan *et al.* Arch. Virol. 1992. 124. 301-319), préparé selon la technique de l'exemple 2, et avec les oligonucléotides suivants: CD064 (39 mer) (SEQ ID N° 23) 5'TTCTTGCGGCCGCCATGGCAAGACGAGCTCGCAGACCGA 3' 
CD065 (38 mer) (SEQ ID N° 24)
5'TTCTTGCGGCCGCTCAGGGCTGCGTCCCCCAGTACATG 3'
 pour isoler le gène codant pour la protéine de la capside VP1 du CAV sous la forme d'un fragment Notl-Notl. Après purification, le produit de PCR de 1377 pb a été digéré par *Not*I pour isoler un fragment Notl-Notl de 1359 pb. Ce fragment a été ligaturé avec le vecteur pVR1012 (exemple 6), préalablement digéré avec *Notl,* pour donner le plasmide pAB054 (6274 pb) qui contient le gène CAV VP1 dans la bonne orientation par rapport au promoteur (Figure N° 16).

### Exemple 17 : Construction du plasmide pAB055 (gène CAV VP2)

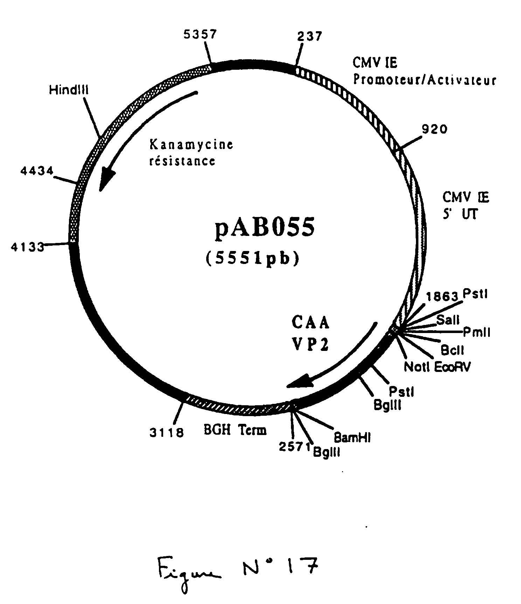

Une réaction de PCR a été réalisée avec l'ADN génomique du virus de l'anémie du poulet (CAV) (Souche Cuxhaven-1) (B. Meehan *et al.* Arch. Virol. 1992. 124. 301-319), préparé selon la technique de l'exemple 2, et avec les oligonucléotides suivants: 
CD066 (39 mer) (SEQ ID N° 25)
5'TTCTTGCGGCCGCCATGCACGGGAACGGCGGACAACCGG 3'
 
AB105 (32 mer) (SEQ ID N° 26)
5'CGCGGATCCTCACACTATACGTACCGGGGCGG 3'
 pour isoler le gène codant pour la protéine VP2 du virus CAV sous la forme d'un fragment Notl-BamHI. Après purification, le produit de PCR de 674 pb a été digéré par *Not*I et *Bam*HI pour isoler un fragment NotI-BamHI de 659 pb. Ce fragment a été ligaturé avec le vecteur pVR1012 (exemple 6), préalablement digéré avec *Not*I et *Bam*HI, pour donner le plasmide pAB055 (5551 pb) (Figure N° 17).

### Exemple 18 : Construction du plasmide pAB076 (gène ILTV gB)

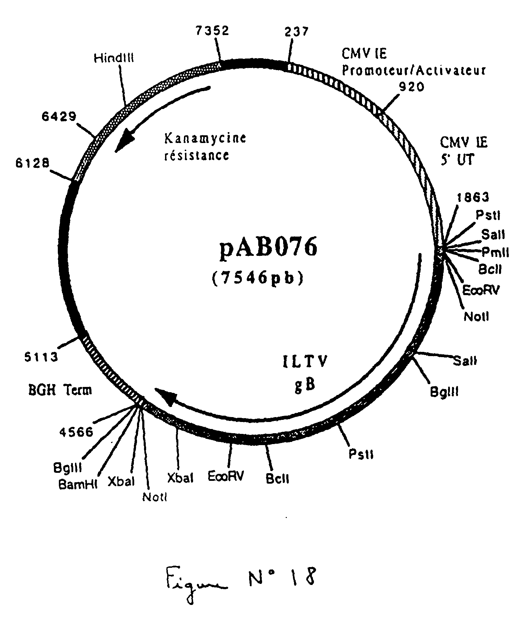

Une réaction de PCR a été réalisée avec l'ADN génomique du virus de la laryngotrachéite infectieuse du poulet (ILTV) (Souche SA-2) (K. Kongsuwan *et al.* Virology. 1991. 184. 404-410), préparé selon la technique de l'exemple 2, et avec les oligonucléotides suivants: 
AB140 (38 mer) (SEQ ID N° 27)
5'TTCTTGCGGCCGCCATGGCTAGCTTGAAAATGCTGATC 3'
 
AB141 (36 mer) (SEQ ID N° 28)
5'TTCTTGCGGCCGCTTATTCGTCTTCGCTTTCTTCTG 3'
 pour isoler le gène codant pour la glycoprotéine gB du virus ILTV sous la forme d'un fragment Notl-Notl. Après purification, le produit de PCR de 2649 pb a été digéré par *Not*l pour isoler un fragment Notl-Notl de 2631 pb. Ce fragment a été ligaturé avec le vecteur pVR1012 (exemple 6), préalablement digéré avec *Not*l, pour donner le plasmide pAB076 (7546 pb) qui contient le gène ILTV gB dans la bonne orientation par rapport au promoteur (Figure N° 18).

### Exemple 20 : Construction du plasmide pAB089 (gène ILTV gD)

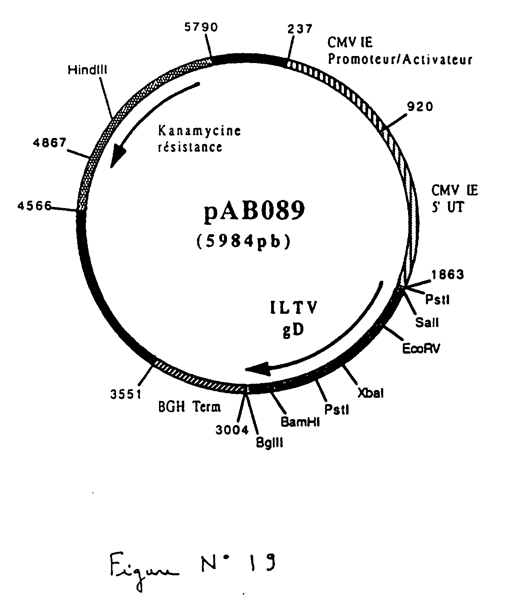

Une réaction de PCR a été réalisée avec l'ADN génomique du virus de la laryngotrachéite infectieuse du poulet (ILTV) (Souche SA-2) (M. Johnson *et al.* 1994. N° d'accès séquence sur Genbank = L31965), préparé selon la technique de l'exemple 2, et avec les oligonucléotides suivants: 
AB164 (33 mer) (SEQ ID N° 29)
5'CCGGTCGACATGGACCGCCATTTATTTTTGAGG 3'
 
AB165 (33 mer) (SEQ ID N° 30)
5'GGAAGATCTTTACGATGCTCCAAACCAGTAGCC 3'
 pour isoler le gène codant pour la glycoprotéine gD du virus ILTV sous la forme d'un fragment Sall-BgIII. Après purification, le produit de PCR de 1134 pb a été digéré par *Sal*I et *Bgl*II pour isoler un fragment Sall-BgIII de 1122 pb. Ce fragment a été ligaturé avec le vecteur pVR1012 (exemple 6), préalablement digéré avec *Sal*I *et Bgl*II, pour donner le plasmide pAB089 (5984 pb) (Figure N° 19).

### Exemple 21 : Construction du plasmide pAB086 (gène AEV env)

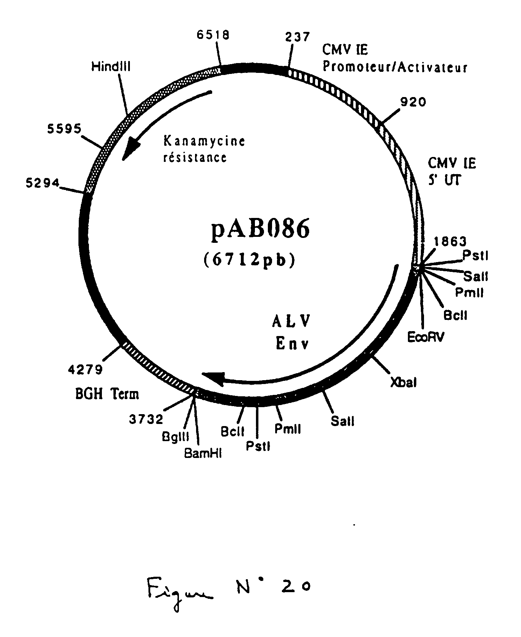

Une réaction de RT-PCR selon la technique de l'exemple 5 a été réalisée avec l'ARN génomique du virus de l'encéphalomyélite aviaire (AEV) (Type C) (E. Bieth *et al.* Nucleic Acids Res. 1992. 20. 367), préparé selon la technique de l'exemple 3, et avec les oligonucléotides suivants: 
AB160 (54 mer) (SEQ ID N° 31)
5'TTTGATATCATGGAAGCCGTCATTAAGGCATTTCTGACTGGATACCCTGG
GAAG 3'
 
AB161 (31 mer) (SEQ ID N° 32)
5'TTTGGATCCTTATACTATTCTGCTTTCAGGC 3'
 pour isoler la séquence codant pour la glycoprotéine Env du virus AEV sous la forme d'un fragment EcoRV-BamHI. Après purification, le produit de RT-PCR de 1836 pb a été digéré par *Eco*RV et *Bam*HI pour isoler un fragment EcoRV-BamHI de 1825 pb. Ce fragment a été ligaturé avec le vecteur pVR1012 (exemple 6), préalablement digéré avec EcoRV et BamHI, pour donner le plasmide pAB086 (6712 pb) (Figure N° 20).

### Exemple 22 : Construction du plasmide pAB081 (gène AEV gag/pro)

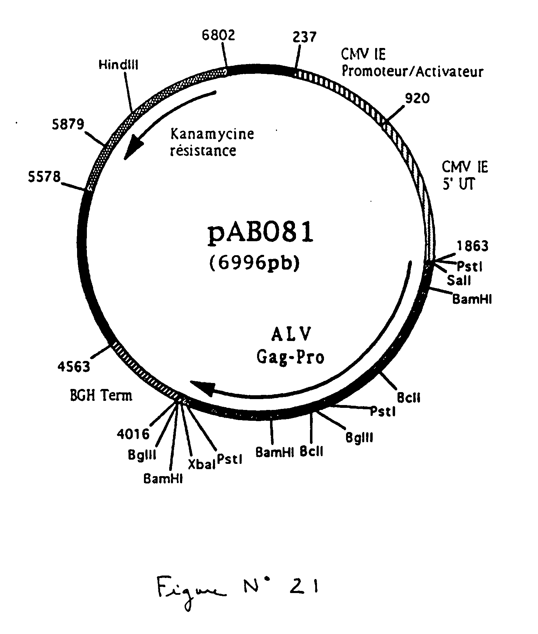

Une réaction de RT-PCR selon la technique de l'exemple 5 a été réalisée avec l'ARN génomique du virus de l'encéphalomyélite aviaire (AEV) (Type C) (E. Bieth *et al.* Nucleic Acids Res. 1992. 20. 367), préparé selon la technique de l'exemple 3, et avec les oligonucléotides suivants: 
AB150 (31 mer) (SEQ ID N° 33)
5'ACGCGTCGACATGGAAGCCGTCATTAAGGTG 3'
 
AB151 (32 mer) (SEQ ID N° 34)
5'TGCTCTAGACTATAAATTTGTCAAGCGGAGCC 3'
 pour isoler la séquence codant pour les protéines Gag et Pro du virus AEV sous la forme d'un fragment SalI-XbaI. Après purification, le produit de RT-PCR de 2125 pb a été digéré par *Sal*I et *Xba*I pour isoler un fragment Sall-Xbal de 2111 pb. Ce fragment a été ligaturé avec le vecteur pVR1012 (exemple 6), préalablement digéré avec *Sal*I et *Xba*I, pour donner le plasmide pAB081 (6996 pb) (Figure N° 21).

### Exemple 23 : Construction du plasmide pAB082 (gène Pneumovirus G)

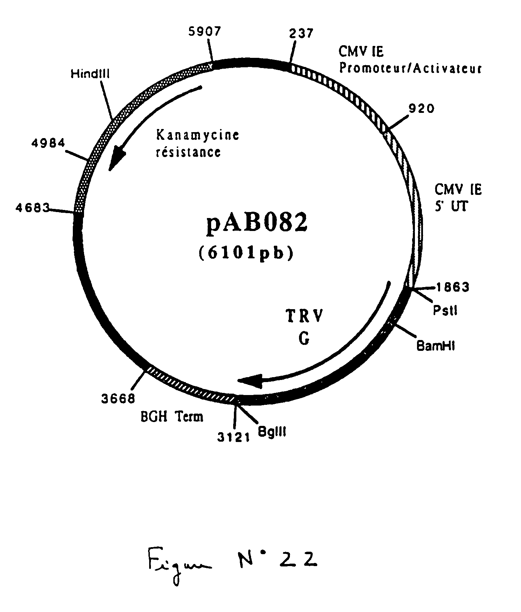

Une réaction de RT-PCR selon la technique de l'exemple 5 a été réalisée avec l'ARN génomique du virus de la rhinotrachéite de la dinde (TRV) (Souche 2119) (K. Juhasz *et al.* J. Gen. Virol. 1994. 75. 2873-2880), préparé selon la technique de l'exemple 3, et avec les oligonucléotides suivants: 
AB152 (32 mer) (SEQ ID N° 35)
5'AAACTGCAGAGATGGGGTCAGAGCTCTACATC 3'
 
AB153 (31 mer) (SEQ ID N° 36)
5'CGAAGATCTTTATTGACTAGTACAGCACCAC 3'
 pour isoler le gène codant pour la glycoprotéine G du virus TRV sous la forme d'un fragment PstI-BglII. Après purification, le produit de RT-PCR de 1265 pb a été digéré par *Pst*I et *Bgl*II pour isoler un fragment PstI-BglII de 1249 pb. Ce fragment a été ligaturé avec le vecteur pVR1012 (exemple 6), préalablement digéré avec *Pst*l et *Bgl*II, pour donner le plasmide pAB082 (6101 pb) (Figure N° 22).

### Exemple 24 : Construction du plasmide pAB077 (gène Peste aviaire HA souche H2N2)

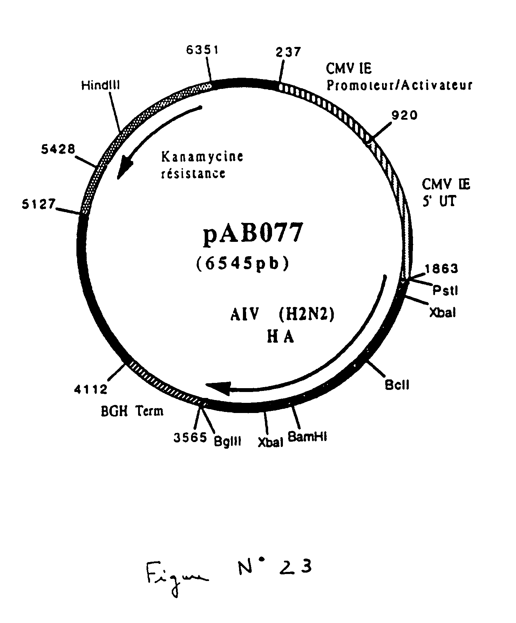

Une réaction de RT-PCR selon la technique de l'exemple 5 a été réalisée avec l'ARN génomique du virus de la peste aviaire (AIV) (Souche H2N2 Postdam) (J. Schäfer *et al.* Virology. 1993. 194. 781-788), préparé selon la technique de l'exemple 3, et avec les oligonucléotides suivants: 
AB142 (33 mer) (SEQ ID N° 37)
5'AAACTGCAGCAATGGCCATCATTTATCTAATTC 3'
 
AB143 (31 mer) (SEQ ID N° 38)
5'CGAAGATCTTCATATGCAGATTCTGCATTGC 3'
 pour isoler le gène codant pour la glycoprotéine HA du virus de la peste aviaire (souche H2N2) sous la forme d'un fragment Pstl-BgIII. Après purification, le produit de RT-PCR de 1709 pb a été digéré par *Pst*I et *Bgl*II pour isoler un fragment Pstl-BgIII de 1693 pb. Ce fragment a été ligaturé avec le vecteur pVR1012 (exemple 6), préalablement digéré avec *Pst*I et *Bgl*II, pour donner le plasmide pAB077 (6545 pb) (Figure N° 23).

### Exemple 25 : Construction du plasmide pAB078 (gène Peste aviaire HA souche H7N7)

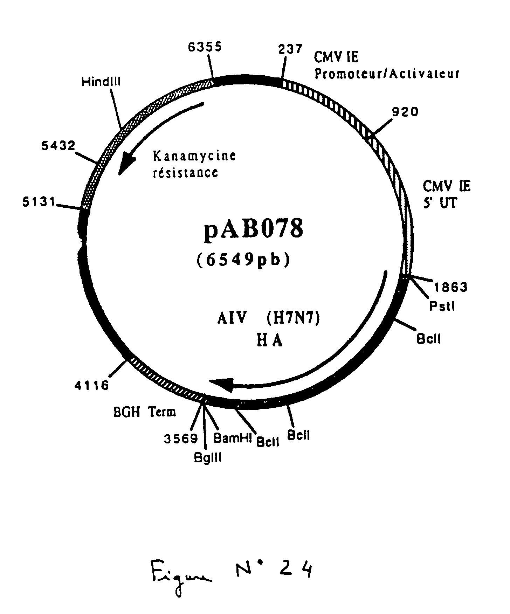

Une réaction de RT-PCR selon la technique de l'exemple 5 a été réalisée avec l'ARN génomique du virus de la peste aviaire (AIV) (Souche H7N7 Leipzig) (C. Rohm *et al.* Virology. 1995. 209. 664-670), préparé selon la technique de l'exemple 3, et avec les oligonucléotides suivants: 
AB144 (31 mer) (SEQ ID N° 39)
5'AAACTGCAGATGAACACTCAAATCCTGATAC 3'
 
AB145 (31 mer) (SEQ ID N° 40)
5'TTTGGATCCTTATATACAAATAGTGCACCGC 3'
 pour isoler le gène codant pour la glycoprotéine HA du virus de la peste aviaire (souche H7N7) sous la forme d'un fragment Pstl-BamHI. Après purification, le produit de RT-PCR de 1707 pb a été digéré par *Pst*l et *Bam*HI pour isoler un fragment Pstl-BamHI de 1691 pb. Ce fragment a été ligaturé avec le vecteur pVR1012 (exemple 6), préalablement digéré avec *Pst*I et *Bam*HI, pour donner le plasmide pAB078 (6549 pb) (Figure N° 24).

### Exemple 26 : Construction du plasmide pAB088 (gène Peste aviaire NP souche H1N1)

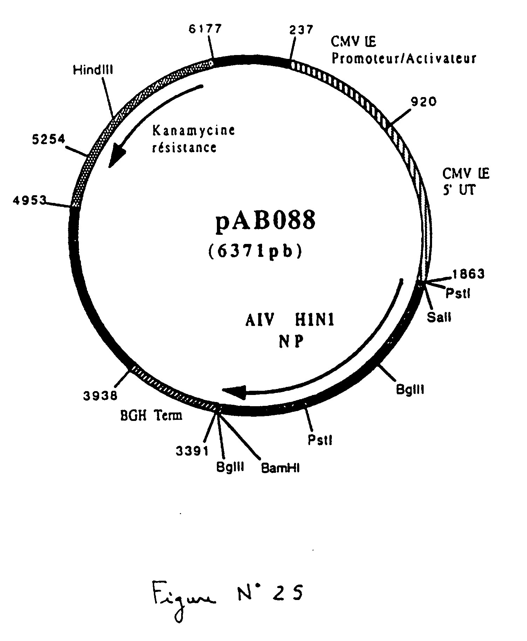

Une réaction de RT-PCR selon la technique de l'exemple 5 a été réalisée avec l'ARN génomique du virus de la grippe aviaire (AIV) (Souche H1 N1 Bavaria) (M. Gammelin *et al.* Virology. 1989. 170. 71-80), préparé selon la technique de l'exemple 3, et avec les oligonucléotides suivants: 
AB156 (32 mer) (SEQ ID N° 41)
5'CCGGTCGACATGGCGTCTCAAGGCACCAAACG 3'
 
AB158 (30 mer) (SEQ ID N° 42)
5'CGCGGATCCTTAATTGTCATACTCCTCTGC 3'
 pour isoler le gène codant pour la nucléoprotéine NP du virus de la grippe aviaire sous la forme d'un fragment SalI-BamHI. Après purification, le produit de RT-PCR de 1515 pb a été digéré par *Sal*I et *Bam*HI pour isoler un fragment SalI-BamHI de 1503 pb. Ce fragment a été ligaturé avec le vecteur pVR1012 (exemple 6), préalablement digéré avec *Sal*I et *Bam*HI, pour donner le plasmide pAB088 (6371 pb) (Figure N° 25).

### Exemple 27 : Construction du plasmide pAB079 (gène-Peste aviaire N souche H7N1)

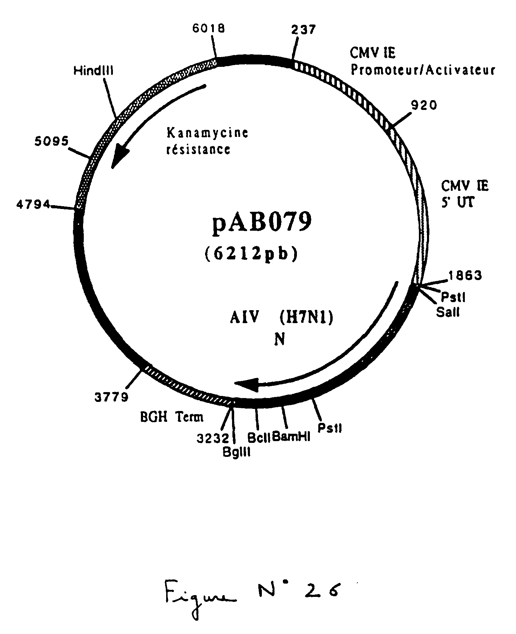

Une réaction de RT-PCR selon la technique de l'exemple 5 a été réalisée avec l'ARN génomique du virus de la peste aviaire (AIV) (Souche H7N1 Rostock) (J. Mc Cauley 1990. N° d'accès séquence sur Genbank = X52226), préparé selon la technique de l'exemple 3, et avec les oligonucléotides suivants: 
AB146 (35 mer) (SEQ ID N° 43)
5'CGCGTCGACATGAATCCAAATCAGAAAATAATAAC 3'
 
AB147 (31 mer) (SEQ ID N° 44)
5'GGAAGATCTCTACTTGTCAATGGTGAATGGC 3'
 pour isoler le gène codant pour la glycoprotéine N du virus de la peste aviaire (souche H7N1) sous la forme d'un fragment SalI-BglII. Après purification, le produit de RT-PCR de 1361 pb a été digéré par *Sal*I et *Bgl*II pour isoler un fragment SalI-BglII de 1350 pb. Ce fragment a été ligaturé avec le vecteur pVR1012 (exemple 6), préalablement digéré avec *Sal*I et *Bgl*II, pour donner le plasmide pA8079 (6212 pb) (Figure N° 26).

### Exemple 28 : Préparation et purification des plasmides

Pour la préparation des plasmides destinés à la vaccination des animaux, on peut utiliser toute technique permettant d'obtenir une suspension de plasmides purifiés majoritairement sous forme superenroulée. Ces techniques sont bien connues de l'homme de l'art. On peut citer en particulier la technique de lyse alcaline suivie de deux ultracentrifugations successives sur gradient de chlorure de césium en présence de bromure d'éthidium telle que décrite dans J. Sambrook *et al.* (*Molecular Cloning: A Laboratory Manual,* 2^{nd} Edition, Cold Spring Harbor Laboratory. Cold Spring Harbor. New York. 1989). On peut se référer également aux demandes de brevet PCT WO 95/21250 et PCT WO 96/02658 qui décrivent des méthodes pour produire à l'échelle industrielle des plasmides utilisables pour la vaccination. Pour les besoins de la fabrication des vaccins (voir exemple 17), les plasmides purifiés sont resuspendus de manière à obtenir des solutions à haute concentration ( > 2 mg/ml) compatibles avec le stockage. Pour ce faire, les plasmides sont resuspendus soit en eau ultrapure, soit en tampon TE (Tris-HCl 10 mM; EDTA 1 mM, pH 8,0).

### Exemple 29 : Fabrication des vaccins associés

Les divers plasmides nécessaires à la fabrication d'un vaccin associé sont mélangés à partir de leurs solutions concentrées (exemple 16). Les mélanges sont réalisés de telle manière que la concentration finale de chaque plasmide corresponde à la dose efficace de chaque plasmide. Les solutions utilisables pour ajuster la concentration finale du vaccin peuvent être soit une solution NACl à 0,9 %, soit du tampon PBS.

Des formulations particulières telles que les liposomes, les lipides cationiques, peuvent aussi être mises en oeuvre pour la fabrication des vaccins.

### Exemple 30 : Vaccination des poulets

Les poulets sont vaccinés avec des doses de 10, 50 ou 100 *µ*g par plasmide. Les injections peuvent être réalisées à l'aiguille par voie intramusculaire. Les sites d'injection sont le bréchet (pour les poulets de plus de 2 semaines) et la cuisse (pour les poulets âgés d'1 jour et plus). Dans ce cas, les doses vaccinales sont administrées sous un volume de 0,1 à 0,3 ml.

Chez le poulet adulte (âgé de plus de 20 semaines), les injections sont également réalisées par voie intramusculaire en utilisant un appareil d'injection à jet liquide (sans aiguille) qui a été spécifiquement conçu pour la vaccination des poulets (par exemple, appareil AVIJET). Dans ce cas, le volume injecté est de 0,3 ml. L'injection peut être effectuée au bréchet ou au niveau de la cuisse. De même, chez le poulet adulte, les injections peuvent être réalisées à l'aiguille par voie intramusculaire, au bréchet ou à la cuisse, sous un volume de 0,3 ml. L'injection des vaccins plasmidiques peut également se faire *in ovo.* Dans ce cas, des formulations particulières, telles que citées dans l'exemple 29 peuvent être utilisées. Le volume injecté dans l'oeuf embryonné de 18 jours est compris entre 50 *µ*l et 200 *µ*l.

### Annexe aux documents de la demande - listage des séquences déposé ultérieurement.

## Revendications

1. Formule de vaccin aviaire comprenant un plasmide qui contient le gène de la protéine VP2 du virus de la maladie de Gumboro et un véhicule approprié.

2. Formule de vaccin selon la revendication 1, telle que ledit plasmide comprend en outre un promoteur choisi dans le groupe constitué par promoteur CMV-IE, promoteur précoce de SV40, promoteur tardif de SV40, promoteur LTR du virus du sarcome de Rous, promoteur propre de VP2, promoteur d'un gène de cytosquelette.

3. Formule de vaccin selon la revendication 2, telle que ledit promoteur est le promoteur de CMV-IE.

4. Formule de vaccin selon l'une quelconque des revendications 1 ou 2, sous forme d'un volume de dose compris entre 0.1 et 1 mL, préférentiellement entre 0.3 et 0.5 mL.

5. Formule de vaccin selon l'une quelconque des revendications 1-4, comprenant une dose comprise entre 10 ng et 1 mg, préférentiellement entre 100 ng et 500 µg, plus préférentiellement entre 0.1 µg et 50 µg dudit plasmide.

6. Formule de vaccin selon l'une quelconque des revendications 1-5, comprenant en outre un vaccin entier vivant, vaccin entier inactivé, un vaccin recombinant, ou un vaccin de sous-unité contre une autre pathologie.

7. Kit de vaccination aviaire comprenant une formule de vaccin selon l'une quelconque des revendications 1-6, et un premier vaccin choisi dans le groupe constitué par vaccin entier vivant, vaccin entier inactivé, un vaccin recombinant, et un vaccin de sous-unité.

8. Utilisation d'une formule de vaccin selon l'une quelconque des revendications 1-6 pour la préparation d'un vaccin aviaire destiné à vacciner des animaux primo-vaccinés au moyen d'un premier vaccin choisi dans le groupe constitué par vaccin entier vivant, vaccin entier inactivé, un vaccin recombinant, et un vaccin de sous-unité.

9. Procédé de préparation d'une formule de vaccin selon l'une quelconque des revendications 1-6.
